# FASCICULE DE BREVET EUROPEEN

(11) **EP 2 736 449 B1**
(45) Date de publication et mention de la délivrance du brevet: **12.08.2015**
(21) Numéro de dépôt: 12738570.6
(22) Date de dépôt: 05.07.2012
(51) Int. Cl.: A61F 2/04, A61F 5/00

(54) **DISPOSITIF CHIRURGICAL D'ANCRAGE CONTROLE DANS L'INTESTIN**
CHIRURGISCHE VORRICHTUNG ZUR GESTEUERTEN VERANKERUNG IM DARM
SURGICAL DEVICE FOR CONTROLLED ANCHORING IN THE INTESTINE

(30) Priorité: 25.07.2011 FR 1156771
(43) Date de publication de la demande: 04.06.2014
(73) Titulaire: Khosrovaninejad, Charam, 84210 Pernes Les Fontaines (FR); Medical Innovation Developpement, 69570 Dardilly (FR)
(72) Inventeur: KHOSROVANINEJAD, Charan, 84210 Pernes Les Fontaines (FR)
(74) Mandataire: Domange, Maxime
(86) Numéro de dépôt international: PCT/FR2012/051576
(87) Numéro de publication internationale: WO 2013/014353

(56) Documents cités:
- WO-A1-03/094785
- WO-A1-2010/092291
- WO-A1-2011/085234
- US-A1- 2008 167 606

## Description

La présente invention concerne un dispositif chirurgical d'ancrage sur la muqueuse de la paroi interne de l'intestin comprenant un élément d'ancrage comprenant au moins un premier élément longitudinal creux semi-rigide, définissant une paroi de révolution autour d'un axe longitudinal comprenant une partie courante multi-perforée sensiblement cylindrique à section sensiblement circulaire dénommée première paroi, ledit élément d'ancrage étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente ladite première paroi présentant un diamètre que l'on peut faire varier de façon contrôlée.

Plus particulièrement, la présente invention concerne un dispositif d'ancrage dont l'ancrage peut être modifié de façon contrôlée.

Des éléments d'ancrage dans l'intestin sont connus notamment des dispositifs appelés « prothèses entérales » ou « stents digestifs » présentant une dite première paroi formée plus particulièrement encore d'un maillage de fils spiralés, de préférence en nitinol, présentant des propriétés de variation de diamètre contrôlée selon la température comme décrit ci-après, dont le diamètre externe peut varier de façon contrôlée entre :
- un premier diamètre externe minimal en dite position radiale rétractée de ladite première paroi, de préférence d'au plus 10mm, et
- un premier diamètre externe maximal en dite position d'expansion radiale maximale de ladite première paroi, de préférence de 18 à 45 mm.

Plus particulièrement encore, la présente invention concerne un dispositif chirurgical apte à réaliser la protection temporaire d'une anastomose sur l'oesophage, l'estomac, le jéjunum, le côlon, le rectum ou le canal anal.

Dans WO 2010/092291 ainsi que dans d'autres documents tels que WO 03/094785 et US 2008/0208357, ledit élément d'ancrage est couplé à une gaine souple externe qui s'étend dans la même direction longitudinale que ledit « stent » à l'extérieure dudit « stent ».

Ces dispositifs peuvent être implantés (1) au niveau de l'intestin grêle comme décrit dans WO 03/094785, (2) au niveau de l'oesophage, comme décrit dans US 2008/0208357 pour lutter contre l'obésité, et (3) au niveau du colon, comme décrit dans WO 2010/092291 pour la protection d'anastomose.

Dans d'autres applications, notamment pour éviter une prolifération cancéreuse, on met en oeuvre un dit stent sans dite gaine et uniquement à des fins de protection permanente de la zone recouverte par ledit stent.

Dans tous les cas, ledit élément d'ancrage doit rester ancré de façon temporaire ou permanente contre la paroi de l'intestin avec ladite gaine s'étendant en aval dudit stent. Dans toutes les applications visées ci-dessus, il peut arriver que ledit élément d'ancrage temporaire ou permanent se décolle de la paroi intestinale et migre trop tôt.

En particulier dans l'application de protection temporaire d'une anastomose décrite dans WO 2010/092291, il est prévu que l'élément d'ancrage temporaire commence à migrer à la fin du délai d'iléus postopératoire, estimé en moyenne de 3 à 5 jours, ainsi, du fait que ladite gaine externe présente une longueur couvrant la distance entre l'anastomose et le site d'ancrage en amont d'au moins 50 cm, de préférence au moins un mètre, et que ledit ancrage est donc ancré suffisamment en amont de l'anastomose pour que, lors de la reprise du transit, son temps de migration entre ladite position d'ancrage et ladite anastomose soit au moins 3 jours, de préférence au moins 6 jours.

Ainsi, dans la mesure où la phase de paralysie intestinale postopératoire, dite iléus, dure de 3 à 5 jours, il est ainsi possible de protéger l'anastomose de cette même période additionnée du temps que met ledit élément d'ancrage pour migrer après libération entre son site d'ancrage en amont de l'anastomose et le site de l'anastomose, étant entendu que ce temps de migration dépend de la distance à parcourir entre le site d'ancrage et l'anastomose. En pratique, une distance de 50 cm à 1 m se traduit par un temps de migration de 3 à 6 jours, de sorte qu'au total l'anastomose sera protégée pendant une durée d'au moins 6 à 11 jours après la réalisation de l'anastomose.

Toutefois, il arrive que le délai d'iléus postopératoire soit exceptionnellement raccourci ou au contraire exceptionnellement trop long dans des délais de 24 heures à 4 semaines, de sorte que, soit le dispositif migre à travers anastomose avant que celle-ci n'ait eu le temps de cicatriser ou, au contraire, le dispositif reste ancré trop longtemps dans l'intestin.

Pour respecter un délai de séjour intra-intestinal approprié, il faut donc tenir compte de la motricité spécifique de l'intestin du patient opéré. Plus l'intestin sera actif, la migration du dispositif sera rapide, étant entendu en outre qu'il est fortement recommandé de favoriser l'activité intestinale postopératoire pour améliorer la réhabilitation rapide du patient.

Pour le traitement curatif des fistules anastomotiques après chirurgie de by-pass pour obésité morbide, le brevet américain US 2008/0208357 décrit un dispositif pour la protection de l'anastomose gastro-jéjunale. Selon ce brevet, une gaine protectrice est solidarisée à l'extrémité avale d'un stent. Le stent est positionné dans l'extrémité inférieure de l'oesophage où il doit se maintenir en place grâce à ses caractéristiques seules. La gaine protectrice traverse l'anastomose et se situe dans l'anse jéjunale. Le défaut majeur de ce dispositif consiste dans le risque de migration inopinée du stent, donc le risque de migration du dispositif tout entier dans l'intestin avec au mieux l'obligation d'extraire le dispositif par des manoeuvres endoscopiques, et au pire le risque d'occlusion ou de perforation de l'intestin obligeant à une nouvelle intervention chirurgicale. Or le risque de migration des stents oesophagiens demeure significatif à 20% pour l'ensemble des stents.

De surcroît, le dispositif d'ancrage temporaire et de protection d'anastomose décrit dans la technique antérieure ne peut empêcher la survenue de fistules anastomotiques dont le délai de cicatrisation est nettement plus long que le délai de séjour intra-intestinal du dispositif, en d'autre termes, le dispositif de protection temporaire risque d'être éliminé bien avant la cicatrisation d'une éventuelle fistule. Dans ce cas, le praticien est contraint de réaliser une stomie de protection ce qui diminue considérablement l'intérêt thérapeutique du dispositif. Il résulte de ce qui précède que le comportement d'un dispositif de protection temporaire d'anastomose ou plus généralement d'ancrage dans l'intestin dépend de facteurs dont les variations entre individus peuvent lui conférer un caractère aléatoire, le praticien n'ayant aucun moyen de modifier le comportement dudit élément d'ancrage une fois celui-ci implanté.

Le temps de séjour du dispositif dans l'intestin dépend de multiples facteurs comme les caractéristiques du stent (force radiale d'ouverture, résistance à la compression, adaptabilité aux déformations de l'intestin, géométrie des mailles, recouvrement de sa surface etc.), les propriétés physiques passives des parois de l'intestin (la viscoélasticité), ses propriétés dynamiques (la contractilité), mais aussi la consistance des selles. L'un des facteurs qui régit le comportement du dispositif est la force de friction qui règne entre les parois du stent et l'intestin. Plus grande est la force de friction, plus lente est la mobilité du stent. Or, le coefficient de friction des parois internes de l'intestin est très faible en raison de la présence d'eau, ce qui par conséquent facilite le glissement du stent.

Ceci peut être compensé de plusieurs façons. L'une consiste à augmenter le coefficient de friction des parois externes du stent au moyen d'un film de recouvrement à propriété adhésive. En effet il a été démontré qu'une surface texturée notamment recouverte de micro-spatules peut présenter des propriétés adhésives plus importantes (Buhl S. et al. Humidity influence on the adhesion of biomimetic fibrillar surfaces. International Journal of Materials Research, 2009; 100, n° 8: 1119 - 1126). Néanmoins, cela ne permet pas d'exercer un contrôle direct à volonté et modulable sur le dispositif pour en modifier le comportement. Un procédé consiste à augmenter le diamètre du stent. Mais, en dehors du risque de lésions traumatiques que cela peut engendrer pour les parois de l'intestin, ce procédé n'offre, là encore, aucun contrôle direct sur le comportement du stent. Enfin, il est possible d'utiliser des stents hybrides semi-résorbables dont la force radiale diminue au fur et à mesure de leur résorption (Shomura Y. Composite material stent comprising metallic wire and polylactic acid fibers, and its mechanical strength and retrievability. Acta Radiol. 2009 May;50(4):355-9). Mais, là encore, le processus de résorption n'est régi par aucune action extérieure à l'intestin, et ne permet pas d'exercer un contrôle direct à volonté et modulable sur le dispositif pour en modifier les propriétés d'ancrage.

Dans d'autres indications, les stents digestifs sont utilisés pour remédier aux sténoses digestives. Il s'agit de sténoses oesophagiennes, en particulier celles compliquées de fistule oeso-trachéale, de sténoses gastriques et duodénales, de sténoses coliques et rectales. Un certain nombre de ces sténoses est traité par des stents nus qui permettent de diminuer le risque de migration aux dépends de leur incrustation dans les parois de l'intestin. D'autres sont traitées par des stents recouverts d'un film qui permet de diminuer le risque d'incrustation aux dépends de l'augmentation du risque de migration.

On cherche donc toujours à fournir un dispositif qui permet d'éviter l'incrustation du stent dans les parois de l'intestin, tout en permettant de contrôler le risque de migration inopiné.

Le but de la présente invention est de fournir un dispositif qui permette de contrôler et de modifier à volonté le comportement de l'élément d'ancrage dans l'intestin.

Plus particulièrement, le but de la présente invention est de fournir un nouveau type de dispositif chirurgical, permettant de modifier à volonté le délai de séjour statique, c'est-à-dire le temps écoulé entre la mise en place du dispositif dans l'intestin et le début de sa migration, et/ou le délai de séjour dynamique du dispositif, c'est-à-dire le délai écoulé entre le début de la migration du dispositif et son élimination de l'intestin.

Plus particulièrement encore, le but de la présente invention est de fournir un dispositif dont on puisse ralentir et/ou interrompre la migration en cas de survenue de fistules anastomotiques pour permettre la cicatrisation de ladite fistule avant la migration dudit dispositif au niveau de ladite fistule.

Pour ce faire, la présente invention consiste essentiellement à mettre à profit la viscoélasticité de l'intestin pour attirer sa paroi interne dite la muqueuse, vers la paroi externe du stent. En effet la muqueuse intestinale est souple et élastique tandis que les parois du stent sont relativement rigides. La paroi intestinale peut être attirée (par aspiration) et accolée aux parois externes du stent sous l'effet d'une pression négative dans l'interface stent-muqueuse. Dans ce cas, la force de friction entre l'intestin et le stent s'accroit rapidement et ce de façon significative de par une sorte d'effet ventouse. Inversement, selon la présente invention, cet effet ventouse peut être modifié voire annulé par injection d'air ou d'une solution liquide entre muqueuses intestinales et stent. Par conséquent, la mobilité du stent devient étroitement liée à l'effet ventouse dont la modulation permet alors d'agir sur le comportement du stent dans l'intestin.

Plus précisément, la présente invention fournit un dispositif chirurgical d'ancrage, apte à s'ancrer sur la muqueuse de la paroi interne de l'intestin comprenant un élément d'ancrage temporaire dont l'ancrage peut être modifié de façon contrôlée, comprenant au moins un premier élément longitudinal creux semi-rigide, définissant une paroi de révolution autour d'un axe longitudinal comprenant une partie courante multi-perforée sensiblement cylindrique à section sensiblement circulaire dite première paroi, ledit premier élément longitudinal creux étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre :
- un premier diamètre externe minimal en dite position radiale rétractée de ladite première paroi d'au plus 20 mm, de préférence d'au plus 10 mm, et
- un premier diamètre externe maximal en dite position d'expansion radiale maximale de ladite première paroi, de préférence de 18 à 45 mm, caractérisé en ce qu'au moins une partie, de préférence, toute la longueur, de la surface interne cylindrique de ladite première paroi est doublée d'une couche étanche indépendante formant une gaine interne, seules les extrémités longitudinales de ladite gaine interne étant fixées de manière étanche audit élément d'ancrage à l'aide des premiers moyens de fixation étanche, de préférence un joint annulaire de colle élastomère, à chaque dite extrémité longitudinale de ladite gaine interne, de façon à délimiter une chambre dénommée chambre de dépression entre ladite gaine interne et ladite première paroi, ledit élément d'ancrage temporaire étant couplé à un tube souple ou semi rigide dénommé tube d'injection-aspiration, s'étendant à l'extérieur dudit élément d'ancrage, une extrémité ouverte dudit tube d'injection-aspiration débouchant dans ladite chambre de dépression.

Plus particulièrement, ledit tube d'injection-aspiration est connecté, de préférence de façon réversible à son extrémité longitudinale libre à un embout de raccordement, lui-même connecté de façon réversible ou apte à être connecté de façon réversible avec un dispositif d'injection ou aspiration d'air ou liquide tel qu'une seringue, ledit embout de raccordement comprenant un dispositif d'obturation, de préférence une vanne anti-reflux et un dispositif de témoin de vide apte à indiquer la teneur du vide dans ladite chambre de dépression à l'autre extrémité dudit tube d'injection-aspiration, ledit dispositif témoin étant de préférence un ballonnet.

On comprend que ledit dispositif témoin de la teneur du vide indique la teneur du vide dans ladite chambre de dépression lorsque ledit dispositif chirurgical selon l'invention est en place contre la paroi interne de l'intestin du patient, ce qui permet de suivre et contrôler ou modifier la force de l'ancrage du dispositif selon l'invention contre la paroi de l'intestin.

On comprend que ledit tube d'injection-aspiration est un tube semi rigide notamment en PE ou PP et présente une longueur apte à tenir en forme et s'étendre à l'intérieur de l'intestin depuis un orifice anal ou nasal du patient jusqu'au dit élément d'ancrage, de préférence la longueur dudit tube d'injection-aspiration étant au moins de 20 cm, plus particulièrement de 50 à 150 cm, et l'extrémité libre dudit tube d'injection-aspiration à l'extérieur du patient est raccordée à un dispositif d'aspiration ou injection de fluide gazeux ou liquide, notamment de l'air ou un liquide froid comme explicité ci-après.

On comprend que ladite chambre définit une chambre étanche entre le film étanche de la gaine interne et la paroi intestinale lorsque ledit élément d'ancrage est en position d'expansion maximale et immobilisé par ancrage contre la surface interne de la paroi intestinale. Et, en cas de décollement dudit élément d'ancrage et/ou en cas de migration trop rapide dudit élément d'ancrage à l'intérieur de l'intestin, ladite chambre perd son étanchéité. Mais, on peut alors aspirer de l'air par ledit tube depuis son extrémité libre depuis l'extérieur du patient pour attirer par aspiration la paroi intestinale contre la surface externe de ladite première paroi sur une partie voire sur toute la surface externe de ladite première paroi délimitant ladite chambre pour ralentir la migration de l'élément d'ancrage voire l'immobiliser en rétablissant le vide dans ladite chambre et donc l'étanchéité de ladite chambre.

Inversement, pour provoquer la migration de l'élément d'ancrage et/ou pour accélérer sa migration, on peut injecter de l'air ou une solution liquide dans la chambre de dépression au moyen dudit tube d'injection-aspiration.

En effet, pour les stents en nitinol, la mémoire de forme de cet alliage permet de modifier sa forme et sa rigidité en fonction de la température ambiante. Plus particulièrement, aux températures situées en dessous de 15°, le nitinol devient souple et malléable. Ainsi, l'injection d'une solution liquide froide entre 0° et 15° dans la chambre de dépression permet de rendre malléable le stent et de faciliter sa mobilisation lors du passage à travers l'anastomose ou d'une zone de rétrécissement par exemple.

Par « premiers moyens de fixation étanche », on entend donc que les dits moyens s'étendent annulairement sur tout le périmètre de ladite gaine interne en section transversale à ses extrémités longitudinales et constituent des parois latérales étanches de délimitation de ladite chambre, le cas échéant autour dudit tube d'injection-aspiration traversant lesdits premiers moyens de fixation étanche comme explicité ci-après.

S'agissant dudit élément d'ancrage, de façon connue, on entend par :
- « semi-rigide », que ladite première paroi dudit élément d'ancrage se maintient dans chacune de ses positions rétractées ou en expansion dans une forme donnée définissant une dite surface de révolution, et
- « diamètre externe que l'on peut faire varier de façon contrôlée », que le diamètre de ladite première paroi peut varier de façon déterminée en fonction de conditions de mise en oeuvre du dispositif, que ce soit des paramètres tels que la température, et/ou des moyens mécaniques indépendants coopérant avec ledit élément d'ancrage tel qu'un instrument de pose, notamment un « introducteur », comme explicité ci-après. De préférence, ladite gaine interne est constituée d'un film étanche (c'est-à-dire non perforé) formant une paroi tubulaire souple.

Ce mode de réalisation offre une facilité de fabrication du dispositif mais aussi une plus grande sensibilité et une plus grande homogénéité de la modulation de l'effet ventouse par injection/aspiration d'air ou liquide par ledit tube d'injection-aspiration.

Toutefois, dans un autre mode de réalisation, ladite gaine interne peut être formée par un deuxième élément longitudinal creux semi-rigide définissant une deuxième paroi présentant des propriétés similaires audit premier élément longitudinal creux comme explicité plus loin. Dans ce cas, ledit tube d'injection-aspiration est intercalé entre des dites première paroi multi-perforée et une deuxième paroi étanche ou multi-perforée mais recouverte d'un film étanche sur sa surface externe.

Dans un mode préféré de réalisation, l'extrémité dudit tube d'injection-aspiration débouchant dans ladite chambre est constituée d'une portion de tube multi-perforé s'étendant dans ladite chambre de préférence sensiblement sur toute sa longueur de ladite chambre, de préférence encore sous forme d'une ligne en Z en parcourant sensiblement hélicoïdalement ladite chambre de dépression collé contre la surface interne dudit élément d'ancrage.

On comprend qu'à son extrémité située dans la chambre de dépression le tube d'injection-aspiration est muni de multiples perforations latérales.

Ce mode de réalisation est préféré car il permet de gérer la pression dans ladite chambre de dépression de manière plus uniforme sur toute sa longueur. La forme en Z (ou en zig-zag) du tube d'injection-aspiration permet que celui-ci puisse se replier de manière plus compacte sur lui-même lors de la rétractation du diamètre de ladite première paroi voire de ladite deuxième paroi de l'élément d'ancrage.

Selon une autre caractéristique particulière avantageuse pour la facilité de fabrication du dispositif, l'extrémité dudit tube d'injection-aspiration débouchant dans ladite chambre de dépression traverse de façon étanche un desdits premiers moyens de fixation, de préférence un dit joint annulaire de colle, à une extrémité longitudinale de ladite gaine interne.

Par « traverse de façon étanche », on entend que la liaison entre la surface externe du tube et l'orifice par lequel il traverse lesdits premiers moyens de fixation ou ladite gaine interne, est étanche.

Pour de nombreuses applications notamment de protection d'anastomose, avantageusement, le dispositif comprend en outre une gaine externe à paroi tubulaire constituée d'un film souple fixé audit élément d'ancrage à une extrémité longitudinale dudit élément d'ancrage. De préférence encore, pour des raisons de facilité de fabrication, ladite gaine externe constitue un prolongement de ladite gaine interne s'étendant à l'extérieur dudit élément d'ancrage, en particulier dans la direction longitudinale de celui-ci et, plus particulièrement encore, en aval de celui-ci.

On entend ici par « gaine externe » une gaine qui s'étend à l'extérieur dudit élément d'ancrage. Mais, dans un autre mode de réalisation, la gaine interne et la gaine externe peuvent être constituées de 2 matériaux élastomères différents.

Lesdites gaine interne et gaine externe peuvent être deux parties d'une même gaine.

Dans le cas où le dispositif de l'invention est implanté dans l'oesophage comme décrit dans US 2008/0208357, le tube d'injection-aspiration s'étend à l'extérieur du stent en amont de celui-ci, tandis que ladite gaine externe s'étend toujours en aval du stent. En effet, le tube d'injection-aspiration parcourt la lumière de l'oesophage vers l'oropharynx, passe dans l'une des cavités nasales pour sortir à travers l'orifice nasal correspondant. Le trajet du tube d'injection-aspiration est celui emprunté par le tube d'aspiration naso-gastrique mis en place de façon systématique à travers l'anastomose pour aspirer les secrétions digestives et éviter l'accumulation d'air dans l'estomac durant la phase postopératoire. Le dispositif selon la présente invention permet le maintien du dispositif dans l'oesophage de par l'effet ventouse décrit ci-dessus.

Le dispositif selon la présente invention peut être utilisé aussi après la résection des tiers moyens et inférieurs de l'oesophage, et la résection totale ou partielle de l'estomac pour des pathologies variées et en particulier néoplasiques. Les anastomoses oeso-gastriques ou oeso-coliques intra-thoraciques comportent un risque significatif de fistule dont les conséquences septiques dans le médiastin sont graves et représentent la principale cause de mortalité chez ces opérés.

Dans d'autres modes d'applications, notamment pour les dispositifs de protection temporaires d'anastomose au niveau du côlon pour lequel le tube d'injection-aspiration est introduit par l'orifice anal et non par une cavité nasale ou buccale, ledit tube d'injection-aspiration et ladite gaine externe s'étendent à l'extérieur dudit élément d'ancrage depuis la même extrémité dudit élément d'ancrage et de préférence ledit tube d'injection-aspiration et ladite gaine externe sont solidarisés entre eux.

La solidarisation, notamment par collage, du tube d'injection-aspiration sur la paroi de ladite gaine externe, évite qu'il ne se torsionne sur lui-même et/ou tournent autour l'un de l'autre et ne créent des noeuds ou autre obstacle dans la bonne extension dudit tube et/ou de ladite gaine externe à l'extérieur dudit élément d'ancrage.

De préférence encore, ledit tube d'injection-aspiration est situé à l'extérieur ladite gaine externe, à savoir ici à l'extérieur dans la direction radiale en section transversale.

Ce mode de réalisation est préféré car il permet de manipuler le tube plus facilement que s'il était situé à l'intérieur de ladite gaine externe sans compter la gêne qui résulterait de l'interférence des matières organiques parcourant l'intérieur de ladite gaine externe lorsque le transit intestinal est repris.

Dans une variante, ledit tube d'injection-aspiration est intégré au sein de la paroi tubulaire de ladite gaine externe.

Dans un mode de réalisation avantageux, le dispositif selon l'invention est caractérisé en ce que ledit élément d'ancrage comporte un deuxième élément longitudinal creux semi-rigide, définissant une paroi de révolution autour dudit axe longitudinal (XX) comprenant une partie courante multi-perforée sensiblement cylindrique à section sensiblement circulaire dite deuxième paroi, ladite deuxième paroi étant disposée co axialement à l'intérieur de ladite première paroi, les extrémités longitudinales de ladite deuxième paroi étant fixées de manière étanche à celles de ladite première paroi à l'aide de premiers ou deuxièmes moyens de fixation étanches, de préférence un joint annulaire de colle élastomère à chaque dite extrémité longitudinale de ladite deuxième paroi, ledit deuxième élément longitudinal creux étant réalisé en un matériau lui conférant des propriétés d'élasticité radiale de sorte qu'il peut être comprimé radialement en dite position rétractée et adopter une dite position d'expansion radiale maximale après relâchement de la compression radiale de telle sorte que ledit deuxième diamètre externe de ladite deuxième paroi puisse varier de façon contrôlée entre :
- un deuxième diamètre externe minimal (D2') en position radiale rétractée de ladite deuxième paroi, inférieur audit premier diamètre externe minimal, et
- un deuxième diamètre externe maximal (D2) en position d'expansion radiale maximale de ladite deuxième paroi, inférieur audit premier diamètre externe maximal.

Plus particulièrement, ladite deuxième paroi multi-perforée est intercalée entre ladite gaine interne et ladite première paroi, l'extrémité dudit tube d'injection-aspiration débouchant de préférence dans ladite chambre de dépression entre ladite gaine interne et ladite deuxième paroi.

Dans un mode de réalisation, l'écart entre ledit deuxième diamètre externe maximal et ledit premier diamètre externe maximal étant d'au moins 0,2mm, de préférence de 0,2 à 10 mm, de préférence encore de 1 à 5 mm.

Dans un autre mode de réalisation, ladite deuxième paroi est recouverte de ladite gaine interne, ladite extrémité ouverte, de préférence multi perforée, dudit tube d'injection-aspiration étant intercalée entre les dites première paroi multi-perforée et la deuxième paroi multi-perforée recouverte de ladite gaine, ladite gaine pouvant recouvrir la surface externe ou de préférence la surface interne de ladite deuxième paroi.

Dans un mode de réalisation particulier, ladite première paroi présente :
a) une longueur d'au moins 30 mm, de préférence de 40 à 150 mm, et un diamètre externe que l'on peut faire varier de façon contrôlée entre un diamètre externe minimal en position radiale rétractée de ladite première paroi d'au plus 10 mm, et un diamètre externe maximal en position d'expansion radiale maximale de ladite première paroi, de 18 à 45 mm, de préférence de 20 à 35 mm, et
b) de préférence, au moins une de ses extrémités longitudinales, de préférence encore son extrémité amont, avec une prolongation évasée dénommée collerette, définissant une paroi de révolution autour dudit axe longitudinal à section circulaire de diamètre croissant, de préférence de 5 à 30 mm de longueur, de préférence encore de 15 à 20 mm de longueur, et de diamètre maximal égal à au moins 105% du dit premier diamètre maximal, de préférence de 105 à 150%, notamment avec D0 de 20 à 40 mm.

Ce mode de réalisation est particulièrement avantageux, notamment lorsque la face externe de la première paroi est revêtue d'une couche élastomère multi-perforée, car cette collerette a pour fonction de ralentir la migration du dispositif à l'intérieur de l'intestin même une fois que l'ancrage de celui-ci contre la paroi interne de l'intestin est supprimé dans sa partie longitudinale courante ou surface externe de la première paroi, notamment lors de la reprise du transit intestinal.

Dans un mode préféré de la réalisation, ledit deuxième élément longitudinal creux comporte une deuxième paroi ajustée contre la surface interne de ladite première paroi lesdites première et deuxième parois étant de préférence de même longueur.

Ce mode réalisation est préféré car ledit élément d'ancrage ainsi constitué par un double stent exerce une force radiale accrue de sorte qu'il reste ancré sur la paroi intestinale pendant une période suffisamment longue après la reprise de l'activité intestinale postopératoire. Ceci résulte vraisemblablement du fait que la solidarisation des deux stents rigidifie ledit élément d'ancrage sur son ensemble. D'autre part, l'épaisseur des deux dites première et deuxième paroi ainsi juxtaposées crée un écart suffisant entre ladite gaine interne et la paroi intestinale pour éviter le colmatage des perforations du tube d'injection-aspiration dans ladite chambre de dépression par la gaine interne aspirée contre ladite paroi intestinale comme évoqué ci-dessus.

On comprend que ce mode de réalisation présente l'avantage de maintenir en permanence un espace annulaire de séparation entre ladite gaine interne et ladite paroi intestinale, du fait de l'écart entre les deux première et deuxième parois, ce qui permet de contrôler plus facilement la teneur du vide dans ladite chambre de dépression. En effet, ladite gaine interne étant un film souple étanche, celui-ci peut être difficile à décoller uniformément de la paroi intestinale le cas échéant à cause d'un effet ventouse excessif.

De préférence encore, chaque dit premier et second élément longitudinal creux comporte à une des extrémités longitudinales de chaque dite première et respectivement deuxième paroi une prolongation évasée dénommée collerette, définissant une surface de révolution autour dudit axe longitudinal (XX) à section circulaire de diamètre croissant depuis l'extrémité de ladite première ou respectivement deuxième paroi vers l'extrémité dudit premier ou respectivement deuxième élément longitudinal creux, et de diamètre maximal (D0) égal à au moins 105% dudit premier diamètre maximal (D1), de préférence de 105 à 150%, les deux dits élément longitudinaux creux étant emboités tête-bêche de sorte que ledit élément d'ancrage comporte une dite collerette à chaque extrémité longitudinale.

On comprend que les deux stents sont emboîtés l'un dans l'autre par leurs extrémités sans collerette.

Une telle collerette permet d'obtenir un diamètre plus large sur lune longueur courte donc une meilleure adhérence tout en limitant le risque de perforation de l'intestin.

Shim et collaborateurs ont décrit une technique pour le traitement des sténoses néoplasiques du colon avec la mise en place de 2 stents coaxiaux emboités l'un dans l'autre (Shim CS et al. Double colonic stenting in proximal malignant colonic obstruction. Endospcopy 2004 ;36 :426-431). Il s'agit de 2 stents individuels, mis en place dans la lumière colique, séparément l'un après l'autre, chacun au moyen de son propre introducteur. Moon et collaborateurs ont également rapporté des expériences avec un double stent à base de nitinol (Moon CM. Et al. Comparison of a newly designed double-layered combination covered stent and D-Weave uncovered stent for decompression of obstructive colorectal cancer : a prospective multicenter study Dis Colon Rectum 2010;53:1190-1196). Les 2 stents sont identiques fabriqués à partir d'un fil de nitinol double brin et à mailles ouvertes sans élément de solidarisation entre les 2 stents, ce qui leur confère une déformabilité radiale et longitudinale importante et facilite leur introduction dans le canal opérateur de 3 mm du coloscope. Un film non perforé est intercalé entre les 2 stents. Le stent externe permet de réaliser une bonne incrustation sur la paroi intestinale, tandis que le stent interne est recouvert d'une couche d'un film non-perforé qui empêche la prolifération de la tumeur à l'intérieure du stent interne et préserve donc un canal libre à l'intérieur du stent interne. En revanche, ces double stents de la technique antérieure ne délimitent pas une chambre de dépression selon la présente invention et/ou ne coopèrent pas avec un dit tube d'injection-aspiration, permettant de contrôler depuis l'extérieur les propriétés d'ancrage et/ou de migration du dit élément d'ancrage. D'autre part, les 2 stents selon la présente invention sont de préférence simple brin avec des éléments de solidarisation et quasi sans déformation longitudinale comme explicité plus loin.

Dans un mode de réalisation ladite gaine externe est collée à la face interne de la collerette aval. Dans un autre mode de réalisation la surface interne d'une ou des deux collerettes est recouverte par un film étanche non perforé bio-compatible.

Plus particulièrement encore, ladite gaine externe présente au repos une longueur en aval dudit élément d'ancrage d'au moins 50 cm, de préférence au moins 1 m, et un diamètre externe de 18 à 45 mm, de préférence de 20 à 35 mm.

Ce dispositif est plus particulièrement utile pour réaliser la protection temporaire d'une anastomose sur le côlon, le rectum ou le canal anal.

Dans ce cas, de façon connue, le diamètre externe réduit D'₁ en position radiale rétractée de ladite première paroi permet d'introduire ledit élément d'ancrage dans cette position radiale rétractée, par voie anale puis l'acheminer dans le côlon jusqu'à une position d'ancrage temporaire en amont d'une anastomose sur le côlon, le rectum ou le canal anal. Et, le diamètre externe maximal D1 en position d'expansion radiale maximale de ladite paroi permet que ledit élément d'ancrage reste ancré temporairement, fixé par la force d'expansion radiale, contre la paroi de l'intestin, en l'absence de transit intestinal, et soit apte à être libéré et commencer à migrer lorsque l'intestin se contracte et se dilate lors de ce qu'on appelle « phase de péristaltisme », à la reprise du transit intestinal.

On comprend que ledit élément d'ancrage peut alors adopter:
- un diamètre externe réduit D'1, de ladite première paroi au moins inférieur à celui de l'intestin au repos, de préférence inférieur à 10mm, de manière à être apte à être introduit par voie anale jusqu'en amont d'une anastomose dans l'intestin, et
- un diamètre externe en expansion radiale maximale de ladite première paroi D1 supérieur ou égal à celui de l'intestin au repos de manière à pouvoir être ancré temporairement par appui sur la paroi intestinale par expansion radiale au moins sur une partie dudit élément d'ancrage, et D1 étant toutefois inférieur au diamètre maximale de l'intestin en travail en phase péristaltique de telle sorte que ledit élément d'ancrage reste fixé contre la paroi de l'intestin en l'absence de transit intestinal, notamment pendant la phase de paralysie intestinale post opératoire dite iléus, mais ne soit plus fixé à l'intestin et en pratique commence à migrer lors de la reprise du transit intestinal par glissement le long de la paroi intestinale.

Un moyen classique pour déterminer D1 est le calibrage de la section d'une des extrémités de l'intestin ou moyen d'instruments appelés « bougies », couramment utilisés par les chirurgiens. Un autre moyen pour déterminer D1 est de le considérer comme égal au diamètre de l'agrafeuse circulaire utilisée pour la confection de l'anastomose mécanique le cas échéant. Dans ce cas D1 peut plus particulièrement varier de 18 à 33 mm, correspondant au diamètre externe des agrafeuses actuellement disponibles dans le commerce.

On entend par « extrémité longitudinale », une extrémité dans la direction longitudinale dudit élément d'ancrage ou le cas échéant de ladite gaine externe.

On entend par longueur et diamètre de la gaine externe « au repos », ses longueur et diamètre lorsque son élasticité longitudinale et respectivement radiale ne sont pas sollicitées.

Après la reprise du transit intestinal, le péristaltisme de l'intestin provoque la libération et migration dudit élément d'ancrage ainsi libéré, mais les propriétés d'expansion radiale de l'élément d'ancrage permettent à sa paroi externe de continuer à glisser au contact de la paroi interne de l'intestin et d'obtenir ainsi une étanchéité suffisante pour empêcher le passage du bol fécal entre la paroi externe de l'élément d'ancrage et la paroi interne de l'intestin et ainsi de continuer à protéger l'anastomose. En effet, le bol fécal doit passer impérativement à l'intérieur de la gaine externe et reste bien séparé des parois de l'intestin au niveau de l'anastomose.

Les valeurs ci-dessus de diamètre en expansion radiale de l'élément d'ancrage correspondent à des valeurs légèrement supérieures au diamètre de l'intestin au repos selon les individus, mais inférieure à la valeur 30 à 60 mm de diamètre maximal de l'intestin dilaté lors du transit intestinal. En outre la longueur de ladite partie courante est telle que la surface de contact entre ledit élément d'ancrage et la paroi du côlon combiné à la force d'expansion radiale, sont telles que ledit élément d'ancrage en position d'expansion maximale soit apte à rester fixé temporairement par sa force expansion radiale contre la paroi de l'intestin en amont de l'anastomose et ne migre pas pendant au moins 3 jours de préférence 5 jours après son ancrage en l'absence de transit intestinal. A défaut d'une longueur d'au moins 30mm de partie courante en contact contre le côlon, ledit élément d'ancrage ne pourrait pas rester ancré au moins 3 jours sans avoir recours à des moyens de fixation, nécessitant alors des moyens et/ou interventions pour désactiver ses moyens de fixation et permettre sa migration, ou sans avoir recours à des élément d'ancrage présentant un diamètre d'expansion maximale plus important ce qui pourrait blesser le côlon et de surcroît empêcherait la libération naturelle de l'élément d'ancrage lors de la reprise du transit.

D'autre part, la longueur de la gaine externe est telle que la distance entre l'anastomose et le site d'ancrage en amont peut être d'au moins 20 cm, de préférence au moins 50 cm, et ledit élément d'ancrage peut être ancré dans le côlon suffisamment en amont de l'anastomose pour que lors de la reprise du transit, son temps de migration entre ladite position d'ancrage et ladite anastomose soit d'au moins 3 jours de préférence au moins 6 jours, et que ladite gaine externe protège l'anastomose, voire dépasse de l'orifice anal lorsque ledit élément d'ancrage est en position d'ancrage.

Ainsi, dans la mesure où la phase de paralysie intestinale post opératoire dite iléus dure de 3 à 5 jours, il est ainsi possible de protéger l'anastomose de cette même période additionnée du temps que met ledit élément d'ancrage pour migrer après libération entre son site d'ancrage en amont de l'anastomose et le site de l'anastomose, étant entendu que ce temps de migration dépend de la distance à parcourir entre le site d'ancrage et l'anastomose. En pratique une distance de 50 cm à 1 m se traduit par un temps de migration de 3 à 6 jours, de sorte qu'au total l'anastomose sera protégée pendant une durée d'au moins 6 à 11 jours après la réalisation de l'anastomose.

Avantageusement, lesdites gaine interne et gaine externe (7) sont en matériau synthétique biocompatible, d'épaisseur de paroi de 0,01 à 1 mm, de préférence en matériaux élastomère du type silicone ou polyuréthane de 0,05 à 1 mm d'épaisseur, et de préférence encore présentant des propriétés d'élasticité radiale et longitudinale, et ladite gaine externe au moins présentant des propriétés de mémoire de forme et des propriétés de non collabilité.

On comprend que ladite gaine externe de par sa constitution en élastomère présente des propriétés d'étirabilité radiale et longitudinale similaires à celle de la paroi intestinale, propriétés qui sont celles d'un matériau élastomère en forme de dite gaine externe, celui-ci présentant des propriétés d'élasticité radiale et longitudinale. Ces propriété d'élasticité radiale et longitudinale de la gaine externe sont similaires à celles de la paroi du côlon et permettent que le transit intestinal se fasse correctement dans ladite gaine externe pendant toute la durée de migration de l'élément d'ancrage soit une durée d'au moins 6 à 10 jours.

L'élasticité longitudinale de la gaine externe élastomère peut être plus importante que celle de l'intestin, sans que cela ne pose de difficulté, au contraire elle présente l'avantage de pouvoir être tirée sur la partie de gaine externe en dépassement anal, de manière à la couper et à ce qu'elle se rétracte à l'intérieur en amont dans le rectum.

De par son élasticité radiale, ladite extrémité longitudinale de la gaine externe reste fixée à ladite extrémité dudit élément d'ancrage quel que soit son niveau d'expansion radiale.

D'autre part, les caractéristiques d'épaisseur de la gaine externe combinée à son élasticité lui confèrent une propriété de mémoire de forme. On entend ici par « propriétés de mémoire de forme » que le matériau élastomère constitutif de ladite gaine externe retrouve naturellement sa forme initiale lorsqu'il est déformé par pliage. Comptetenu de la grande longueur de la gaine externe, ces propriétés de mémoire de forme sont importantes, pour que le matériau retrouve naturellement sa forme longitudinale sans créer de blocage du transit en cas de plicatures de la gaine externe lesquelles peuvent effectivement intervenir pendant sa migration après libération de l'élément d'ancrage.

On entend par « propriétés de non collabilité » que le matériau élastomère constitutif de ladite gaine externe présente un coefficient d'adhérence tel que les deux surfaces opposées de paroi interne de la gaine externe ne se collent pas l'une contre l'autre lors de pliement, ceci pour ne créer aucune résistance au passage des gaz et matières.

Ledit élément d'ancrage peut être maintenu en position radiale rétractée avec un instrument dénommé « introducteur » décrit ci- après, l'expansion radiale intervenant après dégagement de l'élément d'ancrage par rapport à l'introducteur.

On comprend aussi que:
- ladite gaine externe présente un diamètre au repos sensiblement au moins égal au dit diamètre externe réduit D'₁ en rétractation radiale dudit élément d'ancrage creux, et inférieur à celui de l'intestin au repos, de préférence ledit diamètre au repos de ladite gaine externe est sensiblement égal à celui de la paroi intestinale au repos, et
- ladite gaine externe s'étend en aval de l'extrémité dudit élément d'ancrage à laquelle elle est fixée sur une longueur correspondant à la distance entre la position d'ancrage en amont de ladite anastomose et une position en aval de préférence jusqu'à l'orifice anal.

Lesdites première et, le cas échéant, deuxième parois dudit élément d'ancrage sont multi-perforées et ajourées, notamment par des pores ou mini perforations.

Dans un mode de réalisation particulier, lesdits premier et deuxième éléments longitudinaux creux d'ancrage temporaires sont des prothèses entérales du type stent.

Ladite première paroi tubulaire peut être recouverte sur au moins sa face externe d'un revêtement multi-perforé, c'est-à-dire ajouré, en matériau synthétique biocompatible, de préférence un élastomère de type silicone ou polyuréthane. Ce revêtement externe est doublement avantageux en ce qu'il permet de faciliter le largage dudit élément d'ancrage par glissement le long de la paroi intestinale à la reprise du transit et pendant l'ancrage protège la paroi intestinale contre laquelle ledit élément longitudinal est en expansion ce qui pourrait risquer de créer une incrustation dans le tissu de la paroi et empêcher le re-largage subséquent, voire créer une perforation de la paroi intestinale.

Ces prothèses entérales du type connu sous la dénomination « stent » sont utilisées dans les tumeurs intestinales depuis une vingtaine d'années, principalement dans le traitement palliatif des sténoses (rétrécissements) tumorales de l'oesophage, du duodénum et du côlon.

De préférence, ledit élément d'ancrage temporaire est constitué de prothèse(s) entérale(s) formée(s) par un maillage ouvert de fils spiralés métalliques ou en matériau plastique, notamment à base de polyéthylène, éventuellement revêtus par une couche d'un matériau synthétique biocompatible qui recouvre lesdites mailles, ledit revêtement étant de préférence en matériau élastomère biocompatible, tel que du silicone.

De façon connue, l'expansion radiale résulte alors du croisement des fils métalliques dont la variation angulaire permet de varier la largeur du losange ou parallélogramme des mailles dudit maillage de fils spiralés.

De préférence et de façon connue, on utilise un stent dont le dessin et la forme du maillage des fils spiralés permettent d'obtenir une variation du diamètre dudit stent avec un minimum de variation de longueur, de préférence pratiquement sans variation de longueur à la compression radiale.

Avantageusement, ledit élément d'ancrage est réalisé en un matériau qui lui confère une dite expansion par élasticité radiale uniquement à température au moins égale à la température ambiante de 20°C, notamment à la température du corps humain, ledit élément d'ancrage étant en dite position radiale rétractée à une température inférieure à ladite température ambiante, de préférence inférieure à 5°C. On comprend que le matériau en forme tubulaire change de diamètre automatiquement en fonction de la température ambiante.

Plus particulièrement encore, ledit élément d'ancrage est constitué de prothèse(s) entérale(s) formée(s) par un maillage de fils spiralés, de préférence en nitinol.

Le nitinol est un alliage métallique présentant des propriétés d'expansion radiale progressive en fonction de la température, à température supérieure ou égale à la température ambiante (25°C), ce qui permet de le conserver sous forme rétractée à température plus froide, notamment à 4°C au stockage. Une fois rétracté à basse température, il reste ainsi rétracté suffisamment de temps pour pouvoir l'acheminer dans l'intestin à l'aide dudit introducteur. Une fois libérée dans l'intestin, la prothèse retrouve progressivement son expansion radiale sous l'effet d'une température ambiante plus élevée, celle du corps humain.

Plus particulièrement, l'épaisseur de ladite première paroi de l'élément d'ancrage est de 0,1 à 1 mm, de préférence de 0,2 à 0,5 mm.

Il s'agit plus particulièrement de l'épaisseur du fil formant ladite première paroi lorsque celle-ci est constituée d'un dit stent formé par maillage de fils spiralés.

Pour satisfaire les propriétés d'expansion radiale de la gaine élastomère, compte tenu des épaisseurs mises en jeu (0,01 à 0,1 mm), il est nécessaire de mettre en oeuvre des compositions élastomères présentant une certaine dureté, ainsi que collabilité (coefficient d'adhérence), mémoire de forme (DRC- degré de déformation rémanente à la compression), dont les fourchettes de valeur sélectionnée sont explicitées ci-après.

Plus particulièrement, ladite gaine externe au moins et de préférence lesdites gaines internes et gaines externes, est ou sont réalisée en matériau polymérique biocompatible à base d'élastomère de préférence du type silicone et/ou polyuréthane présentant les propriétés suivantes :
- une dureté de 5 à 95 Shore A, de préférence de 50 à70 Shore A,
- une déformation rémanente à la compression, ou DRC de 10% à 35%, de préférence de 17% à 23%,
- un coefficient de friction de 0,30 à 0,90, de préférence de 0,35 à 0,45.

On entend par « dureté » l'énergie de déformation élastique d'un élastomère exprimé en Shore A, selon la norme DIN 53505 par exemple.

On entend par « DRC », le degré de déformation résiduelle après libération d'une charge sur un échantillon élastomère après un temps déterminé selon le test de norme DIN 53517 ISO 815B par exemple la propriété de mémoire de forme à la pliure, le matériau reprenant une forme dépliée dès que la compression ayant créé la pliure est relâchée.

Le « coefficient de friction » peut être mesuré selon la norme ASTM D1894, par exemple.

Les valeurs du coefficient de friction mentionné ci-dessus permettent qu'il n'y ait pas d'adhérence des deux faces opposées de la paroi interne de la gaine, l'une contre l'autre pour ne créer aucune résistance au passage des gaz et matières dans la gaine d'une part, et d'autre part pour qu'en cas de pliure la gaine ne se bouche pas en se refermant sur elle-même.

Plus particulièrement encore, ladite gaine est réalisée en matériau polymérique biocompatible à base de silicone comprenant au moins les composés suivants :
- un élastomère de grade ou qualité « LSR » (« Liquid Silicon Rubber ») dans une proportion pondérale de 75 à 95%,
- un élastomère de grade ou qualité « RTV » dans une proportion pondérale de 2,5 à 12.5 %,
- un élastomère de grade ou qualité « gel » dans une proportion pondérale de 2,5 à 12.5 %.

Cette combinaison d'élastomères de différents grades (« LSR »+ « RTV »+ « gel ») est avantageuse car:
- un élastomère de grade ou qualité LSR apporte la résistance à la déchirure.
- un élastomère de grade ou qualité RTV apporte des propriétés d'élasticité radiale et longitudinale, et
- un élastomère de grade ou qualité gel apporte un coefficient d'adhérence réduit (non collabilité).

Dans un mode de réalisation, ledit élément d'ancrage est une prothèse entérale du type stent recouvert sur au moins sa face externe d'un revêtement multi-perforé en matériau synthétique élastomère de type silicone ou polyuréthane, ledit revêtement élastomère étant, le cas échéant, plus souple que le matériau élastomère constitutif de ladite gaine, la liaison entre ladite gaine et ledit élément d'ancrage se faisant par recouvrement dudit élément d'ancrage par ladite gaine sur une partie seulement de la longueur dudit élément d'ancrage.

Des prothèses entérales de type stent recouverts d'élastomère, notamment à base de silicone de ce type sont bien connues et disponibles dans le commerce.

Ainsi, l'élastomère recouvrant le stent présente de plus grandes déformabilités radiale et longitudinale que l'élastomère de la gaine, c'est-à-dire que l'élastomère du revêtement du stent est constitué le cas échéant d'un mélange de différents types de silicone, présentant une teneur pondérale en grade RTV supérieur à celui de l'élastomère de la gaine, tout en restant dans la fourchette mentionnée ci-dessus de 2,5 à 12,5%.

Comme explicité ci-après, ce mode de réalisation permet en exerçant une traction sur la gaine d'induire une réduction du diamètre et une élongation axiale du stent permettant de le désolidariser de l'intestin pour faciliter sa migration à travers l'anastomose lors de la libération dudit élément d'ancrage.

Le coefficient de friction de la surface externe de l'élément d'ancrage peut être augmenté au moyen d'un film de recouvrement à propriété adhésive présentant une surface externe texturée notamment recouverte de micro-spatules, telles que décrit ci-dessus.

Avantageusement encore, on met en oeuvre un dit élément d'ancrage de type stent comportant une prolongation longiligne ou filiforme à son extrémité, appelée de façon connue « lasso » permettant d'effectuer de façon connue de l'homme du métier, éventuellement avec un outil tel qu'un coloscope, une traction sur l'extrémité dudit élément d'ancrage du type stent, afin d'en faciliter la libération et/ou la migration à travers la zone d'anastomose lorsque cela est souhaité de façon à éviter un blocage à ce niveau.

Avantageusement, ledit matériau polymérique de ladite gaine comprend des filaments radio-opaques, notamment de sulfate de baryum, disposés dans la direction longitudinale du tube.

Ces filaments permettent de suivre la migration du tube de la gaine et contrôler son positionnement initial puis sa migration progressive lors de son élimination. D'autre part, le positionnement longitudinal de ces filaments confère à la gaine simultanément une résistance à l'allongement et réduit son élasticité longitudinale qui peut être excessive comme par rapport à celle de l'intestin comme explicité précédemment.

Avantageusement, la gaine est graduée dans le sens croissant en partant de son extrémité amont.

Plus particulièrement, l'introducteur peut être de façon connue constitué d'un tube guide semi rigide, du type cathéter muni à une de ses extrémités d'une poignée et dont le diamètre interne et la longueur permettent d'y maintenir logé ledit élément d'ancrage sous sa forme rétractée et ladite gaine, de préférence déployée longitudinalement.

Pour une introduction par voie anale, de préférence, ledit dispositif comprend en outre un instrument dénommé introducteur comprenant :
- une enveloppe externe tubulaire apte à contenir et maintenir ledit élément d'ancrage comprimé en dite position rétractée et ledit tube d'injection-aspiration à l'intérieur de l'extrémité distale da dite enveloppe externe, et suffisamment long pour contenir en outre ladite gaine et ledit tube d'injection-aspiration, ladite enveloppe externe étant de préférence d'une longueur d'au moins 70 cm, de préférence au moins 100 cm, et
- des moyens pour acheminer l'extrémité distale dudit introducteur depuis l'orifice anal jusqu'au dit site d'ancrage dans l'intestin en amont de l'anastomose, et
- de préférence, des moyens pour dégager ledit élément d'ancrage par rapport à l'enveloppe externe, de préférence encore consistant en un tube butoir comportant une butée à son extrémité distale, en contact le cas échéant avec l'extrémité longitudinale dudit élément d'ancrage, ladite gaine en aval de l'élément d'ancrage entourant ledit tube butoir à l'intérieur de ladite enveloppe externe.

Le retrait de l'enveloppe externe, puis du tube butoir permet de déployer complètement la gaine en aval de l'élément d'ancrage sans qu'aucune manoeuvre supplémentaire du chirurgien ne soit nécessaire pour déployer la gaine.

De préférence encore, le dispositif selon l'invention comprend en outre un tube protecteur comprenant une partie de forme courbe selon la courbure de la concavité sacrée, de rigidité supérieure à celle de l'enveloppe externe de l'introducteur, de diamètre externe et longueur tels que ledit tube protecteur est apte à être introduit par l'orifice anal et s'étendre entre l'orifice anal et jusqu'en amont de ladite anastomose et un diamètre interne et une dite courbure, selon la courbure de la concavité sacrée, tels que ledit tube protecteur est apte à contenir ledit introducteur et à en permettre l'acheminement entre l'orifice anal et ladite anastomose, de préférence un tube protecteur de diamètre externe de 20 à 40 mm et de longueur de 10 à 25 cm.

La présente invention permet de mettre en oeuvre un procédé de traitement chirurgical à l'aide d'un dispositif chirurgical selon l'invention dans lequel on réalise les étapes successives suivantes dans lesquelles:
1) On introduit un dit dispositif chirurgical par voie anale ou nasale, et on l'achemine jusqu'à un site d'ancrage dans l'intestin, ledit élément d'ancrage étant maintenu en dite position radiale rétractée D'₁ et acheminé à l'aide d'un instrument dénommé introducteur, et ledit tube d'injection-aspiration étant d'une longueur au moins égale à la distance entre le site d'ancrage et l'orifice anal ou respectivement nasal.
2) On dégage l'introducteur par rapport audit élément d'ancrage une fois acheminé au niveau du dit site d'ancrage de manière à permettre ainsi que ledit élément d'ancrage adopte une dite position d'ancrage contre la paroi intestinale en dite position d'expansion radiale maximale, et
3) Le cas échéant, on aspire l'air à l'extrémité libre dudit tube d'injection-aspiration à l'extérieur du patient pour empêcher la migration dudit élément d'ancrage et/ou ralentir la migration dudit élément d'ancrage, et
4) Le cas échéant, on injecte de l'air à l'extrémité libre dudit tube d'injection-aspiration et/ou un liquide, notamment un liquide de froid, pour faciliter et/ou accélérer la migration dudit élément d'ancrage dans l'intestin.

A l'étape 1 ci-dessus, ladite gaine externe élastomère est d'une longueur au moins égale à la distance entre le site d'ancrage et l'orifice anal ou d'une longueur d'au moins 10 cm en aval du site d'ancrage oesophagien, de préférence entre 20 et 50 cm.

Ce procédé est utile notamment pour la protection temporaire d'une anastomose sur le gros intestin ou côlon, le rectum ou canal anal, afin de prévenir ou réduire les risques de fistule anastomotique. Dans ce cas, de préférence, la distance entre ladite position d'ancrage en amont de ladite anastomose et ladite anastomose est au moins égale à la distance parcourue en au moins trois jours de préférence au moins cinq jours par ledit élément d'ancrage en migration une fois libéré de ladite paroi intestinale par la reprise du transit intestinal.

Plus particulièrement, la distance entre l'anastomose et le site d'ancrage est d'au moins 20 cm, de préférence au moins 50 cm.

De préférence, on introduit ledit introducteur dans l'orifice anal en le poussant à l'intérieur d'un tube protecteur comprenant une partie de forme courbe selon la courbure de la concavité sacrée, semi rigide, de rigidité supérieure à celle de l'enveloppe externe de l'introducteur, de diamètre externe et longueur, tels que ledit tube protecteur est apte à être introduit par l'orifice anal et s'étendre entre l'orifice anal et jusqu'en amont de ladite anastomose, et un diamètre interne et une dite courbure, selon la courbure de la concavité sacrée, tels que ledit tube protecteur est apte à contenir ledit introducteur et à en permettre l'acheminement entre l'orifice anal et ladite anastomose, celui-ci s'étendant entre l'orifice anal et ladite anastomose, de préférence un tube protecteur de diamètre externe de 20 à 40 mm et de longueur de 10 à 25 cm.

De préférence encore, on récupère ledit élément d'ancrage en migration une fois libéré de ladite paroi intestinale par la reprise du transit intestinal, dans un tube protecteur comprenant une partie de forme courbe selon la courbure de la concavité sacrée semi rigide, de rigidité supérieure à celle de l'enveloppe externe de l'introducteur, de diamètre externe et longueur tels que ledit tube protecteur est apte à être introduit par l'orifice anal et s'étendre entre l'orifice anal et jusqu'en amont de ladite anastomose, et un diamètre interne et une dite courbure, selon la courbure de la concavité sacrée, tels que ledit tube protecteur est apte à contenir ledit introducteur et à en permettre l'acheminement entre l'orifice anal et ladite anastomose, celui-ci s'étendant entre l'orifice anal et ladite anastomose, de préférence un tube protecteur de diamètre externe de 20 à 40 mm et de longueur de 10 à 25 cm.

Les dispositifs d'introducteurs et tubes protecteurs mentionnés ci-dessus ont été décrits de façon plus détaillée dans WO 2010/092291.

Dans un mode préféré de réalisation, notamment lorsque ledit élément d'ancrage comporte une collerette, de préférence une collerette à chaque extrémité, un fil dénommé lasso est accroché en bordure de ladite collerette, de préférence l'un au moins des deux fils étant d'une longueur au moins égale à celle dudit tube d'injection-aspiration et le cas échéant de ladite gaine externe.

Dans le cas d'une protection d'anastomose avec introduction par l'orifice anale, le lasso attaché à la collerette en amont passera à travers la lumière de l'élément d'ancrage et le cas échéant de la gaine externe en aval pour s'extérioriser en aval à travers l'orifice anal, ladite gaine externe étant accrochée à l'extrémité avale de la partie courante dudit élément d'ancrage en amont de la collerette avale, de préférence en continuité de ladite gaine interne.

Dans le cas d'une protection de l'oesophage avec introduction par l'orifice nasal ou buccal, le lasso attaché à la collerette en amont passera à l'extérieure du dit tube d'injection- aspiration en amont de l'élément d'ancrage et sortira en s'extériorisant à travers l'orifice nasal ou buccal tandis que le lasso accroché à la collerette avale passera :
- soit à l'extérieur de l'élément d'ancrage entre la partie courante dudit élément d'ancrage et la muqueuse, puis à l'extérieur dudit tube d'injection-aspiration en amont pour s'extérioriser en amont à travers l'orifice nasal ou buccal, en présence d'une gaine externe en aval,
- soit à travers la lumière de l'élément d'ancrage et à l'extérieur du tube d'injection-aspiration en amont pour s'extérioriser en amont à travers l'orifice nasal ou buccal, en l'absence de gaine externe en aval.

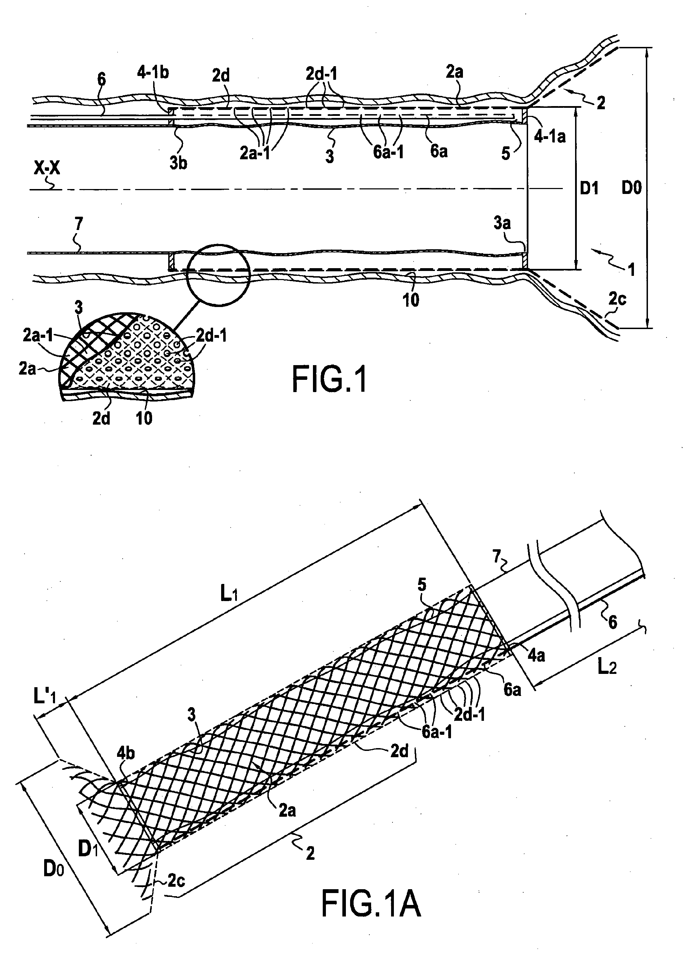

L'intérêt de ces lassos est qu'ils permettent de réduire le diamètre maximal de la collerette et donc l'adhésion de la collerette sur la muqueuse en tirant sur l'extrémité du lasso car les collerettes sont plus intimement ancrées dans la muqueuse que la partie courante de l'élément d'ancrage. D'autres caractéristiques et avantages de la présente invention apparaîtront à la lumière de la description détaillée qui va suivre, faite en référence aux figures 1 à 6 dans lesquelles :
- figure 1 représente de façon schématique en coupe longitudinale un dispositif selon l'invention comportant un seul stent ou dite première paroi et une gaine interne constituée d'un film étanche souple, ladite gaine externe étant constituée d'un même film souple en prolongation de ladite gaine interne,
- la figure 1A est une vue de côté d'un dispositif de la figure 1,
- la figure 2 représente schématiquement un dispositif selon l'invention en coupe longitudinale dans lequel ladite gaine interne recouvre la face externe d'une deuxième paroi formée d'un deuxième stent interne coaxial,
- la figure 3 représente schématiquement en coupe longitudinale un troisième mode de réalisation d'un dispositif selon l'invention comportant également deux stents coaxiaux, ledit tube d'injection-aspiration étant intercalé entre une gaine interne constituée d'un film étanche souple et une dite deuxième paroi du stent interne,
- la figure 4 représente schématiquement un mode de réalisation avec l'élément d'ancrage 2 constitué de deux stents coaxiaux au dites première et deuxième parois 2a, 2b coaxiales, de même diamètre nominal ou sensiblement identique, ajustés l'un dans (et contre) l'autre,
- la figure 5 est une vue en coupe longitudinale d'un dispositif selon l'invention de la figure 3, comprenant le tube d'injection-aspiration multi-perforé selon la figure 5A,
- la figure 5A représente schématiquement en vue de côté selon une coupe longitudinale un mode de réalisation de l'extrémité du tube d'injection-aspiration constitué par une portion de tube multi-perforée, s'étendant selon une forme en Z en parcourant la chambre de dépression sensiblement hélicoïdalement contre une face de ladite première paroi ou de ladite deuxième paroi d'un stent de l'élément d'ancrage,
- la figure 6 représente un dispositif selon l'invention, équipé d'un embout de raccordement 7 coopérant avec une seringue 8, ledit embout 7 comportant une vanne anti-reflux 7a et un ballonnet 7b comportant une tige de raccordement 7c apte à être enfoncée dans l'extrémité du tube d'injection-aspiration 6.

Sur la figure 1 on a représenté un dispositif chirurgical 1 d'ancrage sur la muqueuse de la paroi interne de l'intestin 10 comprenant un élément d'ancrage temporaire 2 constitué par un premier et unique élément longitudinal creux semi-rigide, définissant une paroi de révolution autour d'un axe longitudinal XX comprenant une partie courante multi-perforée sensiblement cylindrique à section sensiblement circulaire dite première paroi 2a, ledit élément d'ancrage 2 étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre :
- un premier diamètre externe minimal D1' en dite position radiale rétractée de ladite première paroi, de préférence d'au plus 10 mm, et
- un premier diamètre externe maximal D1 en dite position d'expansion radiale maximale de ladite première paroi, de préférence de 18 à 45 mm.

Ledit élément longitudinal creux de la première paroi 2a est un stent du type réalisé par maillage spiralé de fils métalliques en nitinol comme schématisé figure 1A, dont l'ancrage peut être modifié de façon contrôlée en fonction de la température. Il présente des propriétés d'expansion radiale contrôlée en fonction de la température. Plus précisément, de par les propriétés du nitinol, ils connaissent une expansion radiale progressive dès qu'ils sont mis à température supérieure à la température d'environ 20°C.

Les perforations multiples 2a-1 de ladite première paroi 2a correspondent aux orifices des mailles réalisés par le maillage spiralé de fils de nitinol.

Les données dimensionnelles quant au diamètre D1' et D1 données ci-dessus correspondent à des dimensions appropriées pour un ancrage du dispositif contre la muqueuse de la paroi interne de l'intestin 10 à différentes positions entre y compris le rectum et l'oesophage.

Sur la figure 1, le stent 2a est revêtu d'une couche de polyuréthane ou de silicone 2d, elle-même multi-perforée en 2d-1 sur la surface externe de 2a. Cette couche 2d protège la paroi intestinale 10 en évitant des incrustations de celle-ci par la surface externe du stent 2a.

Toute la longueur de la surface interne cylindrique de ladite première paroi 2a est doublée d'une couche étanche indépendante formant une gaine interne 3, seules les extrémités longitudinales 3a,3b de ladite gaine interne 3 étant fixées de manière étanche 4a,4b audit élément d'ancrage 2 à l'aide des premiers moyens de fixation étanche 4-1a,4-1b, consistant en un joint annulaire de colle élastomère à chaque dite extrémité longitudinale (3a,3b) de ladite gaine interne.

Sur la figure 1, ladite gaine interne 3 est constituée d'un film étanche formant une paroi tubulaire souple.

Le dispositif selon l'invention comprend en outre une gaine externe 7 à paroi tubulaire constituée d'un film souple fixée audit élément d'ancrage 2 à une extrémité longitudinale dudit élément d'ancrage.

Sur la figure 1, ladite gaine externe 7 constitue un prolongement de ladite gaine interne 3 s'étendant à l'extérieur dudit élément d'ancrage dans la direction longitudinale dudit élément d'ancrage.

Le dispositif selon l'invention comprend en outre un tube souple ou semi-rigide dénommé tube d'injection-aspiration 6 s'étendant à l'extérieur dudit élément d'ancrage 2.

Ladite gaine interne 3 est indépendante de la surface interne de ladite première paroi entre ces deux extrémités 3 et 3b, on comprend que la gaine interne 3 n'est pas tendue d'une manière excessive pour ne pas rigidifier le stent, de sorte que l'écart entre ladite gaine interne 3 et ledit premier diamètre externe maximal D1 de la première paroi 2a est de préférence de 0,2 à 10 mm, de préférence encore de 1 à 5 mm, et l'espace entre ladite gaine interne 3 et ladite première paroi 2a définit une chambre dénommée chambre de dépression 5. Une extrémité ouverte du tube d'injection-aspiration constituée d'une portion de tubes 6a comprenant de multiples perforations 6a-1, s'étend sensiblement sur toute la longueur de ladite chambre 5 dans la direction longitudinale XX du dispositif. Le tube d'injection-aspiration 6 débouche dans ladite chambre de dépression 5 en traversant de façon étanche le joint annulaire de colle élastomère à l'extrémité avale 3b de la gaine 3 lorsque le tube 6 est introduit par l'orifice anal ou le joint à l'extrémité amont 3a de la gaine 3 lorsque le tube 6 est introduit par l'orifice oral.

Ledit tube d'injection-aspiration 6 et ladite gaine externe 7 s'étendent à l'extérieur dudit élément d'ancrage depuis la même extrémité avale dudit élément d'ancrage et de préférence ledit tube d'injection-aspiration (6) et ladite gaine externe (7) sont collés l'un à l'autre à l'extérieur de ladite gaine externe 7 dans le cas d'une introduction par voie anale.

Le tube d'injection-aspiration 6 sert à injecter ou aspirer de l'air ou un liquide dans la chambre 5 pour aspirer ou décoller la paroi intestinale 10 de la face externe du stent 2a, et plus généralement pour modifier la caractéristique d'ancrage du stent 2a par rapport à la paroi intestinale 10. La couche 2d est perforée en 2d-1 pour permettre le passage de l'air ou du liquide.

Sur la figure 1, la portion 6a du tube d'injection-aspiration 6 à l'intérieur de la chambre 5 peut être collée sur la face interne de la première paroi 2a ou sur la face externe de ladite gaine externe.

Dans tous les modes de réalisation, ladite première paroi 2a présente:
a) une longueur L1 d'au moins 30 mm, de préférence de 40 à 150 mm, et un diamètre externe que l'on peut faire varier de façon contrôlée entre un diamètre externe minimal D1' en position radiale rétractée de ladite première paroi d'au plus 10mm, et un diamètre externe maximal D1 en position d'expansion radiale maximale de ladite première paroi, de 18 à 45 mm, de préférence de 20 à 35 mm, et
b) son extrémité longitudinale amont, avec une prolongation évasée 2c dénommée collerette, définissant une paroi de révolution autour dudit axe longitudinal XX à section circulaire de diamètre croissant, de préférence de 5 à 30mm de longueur L1', de préférence encore de 15 à 20mm de longueur, et de diamètre maximal D0 égal à environ 110% du dit premier diamètre maximal, plus particulièrement de 21 à 37 mm.

Cette collerette 2c a pour fonction de ralentir la migration du dispositif 1 à l'intérieur de l'intestin même une fois que l'ancrage de celui-ci contre la paroi interne 10 de l'intestin est supprimé dans sa partie longitudinale courante ou surface externe de la première paroi 2a,

Dans tous les modes de réalisation, ladite gaine externe 7 présente au repos une longueur L2 en aval dudit élément d'ancrage 2 d'au moins 50 cm, de préférence au moins 1 m, et un diamètre externe de 18 à 45 mm, de préférence de 20 à 35 mm.

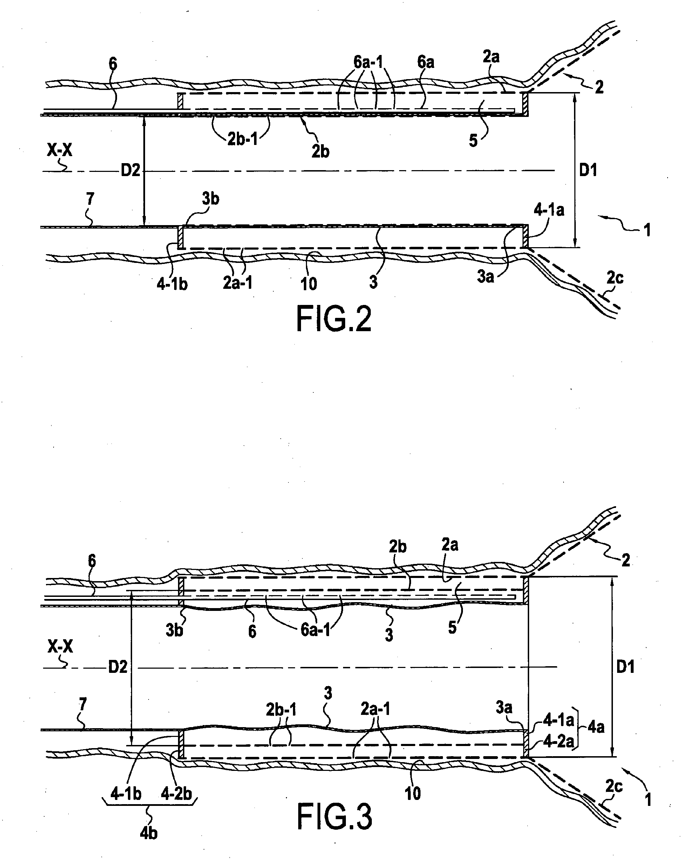

Sur la figure 2, le dispositif selon l'invention comprend un deuxième stent 2b, disposé coaxialement à l'intérieur dudit premier stent 2a, ledit deuxième stent 2b étant constitué d'une autre petite prothèse entérale formée par un maillage de fils spiralés en nitinol dont la surface externe est recouverte d'un film étanche constituant ladite gaine interne 3, ladite deuxième paroi 2b définit une surface de révolution autour dudit axe longitudinal XX comprenant une partie courante sensiblement cylindrique à section sensiblement, ladite deuxième paroi présentant un deuxième diamètre externe inférieur au dit premier diamètre externe de ladite première paroi 2a, les extrémités longitudinales de ladite deuxième paroi 2b étant fixées de manière étanche à celles de ladite première paroi 2a à l'aide de premiers moyens de fixation étanche 4-1a,4-1b consistant en un joint annulaire de colle élastomère 4a,4b à chaque dite extrémité longitudinale de ladite deuxième paroi, de façon à délimiter latéralement ladite chambre de dépression 5 étanche, ledit deuxième élément longitudinal creux étant réalisé en un matériau lui conférant des propriétés d'élasticité radiale de sorte qu'il peut être comprimé radialement en dite position rétractée et adopter une dite position d'expansion radiale maximale après relâchement de la compression radiale de telle sorte que ledit deuxième diamètre externe de ladite deuxième paroi 2b puisse varier de façon contrôlée entre :
- un deuxième diamètre externe minimal D2' en position radiale rétractée de ladite deuxième paroi, inférieur audit premier diamètre externe minimal, et
- un deuxième diamètre externe maximal D2 en position d'expansion radiale maximale de ladite deuxième paroi, inférieur audit premier diamètre externe maximal,
- l'écart entre ledit deuxième diamètre externe maximal D2 et ledit premier diamètre externe maximal D1 étant d'au moins 0,2mm de préférence de 0,2 à 10 mm, de préférence encore de 1 à 5 mm.

Dans ce mode de réalisation de la figure 2, lorsqu'on aspire de l'air depuis l'extrémité extérieure du tube d'injection-aspiration 6, l'aspiration par les perforations 6a1 de la portion de tubes internes dans la chambre de dépression 5 provoque l'aspiration de la paroi intestinal 10 contre la surface externe de la première paroi 2a à travers les mailles 2a-1. Mais, l'espace entre la gaine interne 3 et le stent externe 2a reste sensiblement constant, ce qui permet d'éviter le colmatage des perforations 6a-1 de la portion de tubes 6a et confère donc un fonctionnement plus fiable et plus homogène de contrôle de la teneur en vide à l'intérieure de la chambre de dépression 5. Ceci permet également de contrôler de manière plus fiable et homogène la force de l'ancrage du dispositif 1 contre la paroi interne 10 de l'intestin. Dans le mode de réalisation de la figure 1, il y a un risque que la gaine interne 3 ne vienne se coller contre les perforations 6a-1.

Dans ce mode de réalisation de la figure 2, comme dans le mode de réalisation de la figure 1, le tube d'injection-aspiration 6 traverse le premier moyen de fixation ou joint annulaire de colle élastomère 4-1b à l'extrémité avale de l'élément d'ancrage 2.

Sur la figure 2, la portion 6a du tube d'injection-aspiration 6 à l'intérieur de la chambre 5 peut être collée sur la face externe du film ou gaine interne 3 par-dessus la face externe de la deuxième paroi 2b.

La gaine externe 7 est constituée avantageusement par le prolongement de la direction longitudinale en aval de l'élément d'ancrage de ladite gaine interne 3.

En variante, la gaine externe 7 peut être fixée à son extrémité amont au même joint de colle élastomère 4-1b ou peut être fixée sur la face externe de l'extrémité longitudinale avale de la première paroi 2a par recouvrement de celle-ci sur une courte portion de longueur (non représenté sur les figures).

Dans un mode de réalisation (non-représenté les figures), la gaine 7 à son extrémité amont peut aussi recouvrir la surface externe deuxième paroi 2b constituant ainsi ladite gaine interne 3 ou une partie seulement.

Sur la figure 3, on a représenté un troisième mode de réalisation dans lequel ladite gaine interne 3 est appliquée en vis-à-vis de la surface interne du stent interne ou deuxième paroi 2b, c'est-à-dire à l'intérieur de celle-ci et collée contre celle-ci à ses extrémités longitudinales. Ainsi, on ménage encore une chambre de dépression 5 présentant un écart radial sensiblement permanent en position d'expansion radiale de la première paroi 2a, du fait qu'on met en oeuvre une deuxième paroi multi-perforée 2b comme dans le mode de réalisation de la figure 2 décrit ci-dessus, constituée d'un stent de plus petit diamètre. Le deuxième stent ou stent interne 2b définit une dite deuxième paroi multi-perforée 2b intercalée entre ladite gaine interne 3 et ladite première paroi 2a. Et, l'extrémité dudit tube d'injection-aspiration 6a débouche dans ladite chambre de dépression 5 entre ladite gaine interne 3 et ladite deuxième paroi 2b, ladite gaine interne 3 étant, comme dans la figure 1, constituée par la prolongation en amont de ladite gaine externe 7 constituée d'une paroi tubulaire souple.

Dans le mode de réalisation de la figure 3, la portion 6a de tube d'injection-aspiration 6 intercalée entre la gaine interne 3 et la deuxième paroi 2b à l'intérieur de la chambre 5 peut être collée contre la face interne de la deuxième paroi 2b. Et, lorsque l'on réalise l'aspiration de l'air à travers les ouvertures 6a-1 de la portion de tube 6a dans la chambre 5, la gaine souple 3 est aspirée contre le tube 6a et la paroi intestinale 10 est aspirée contre la face externe du stent externe 2a, mais l'espace entre les deux stents coaxiaux 2a et 2b est maintenu constant, comme décrit ci-dessus, d'un écart d'au moins 0,2 à 10 mm, de préférence de 1 à 5 mm, en position radiale d'extension desdits stents. Il en résulte le même avantage que dans le mode de réalisation de la figure 2 en termes de facilité, homogénéité et fiabilité du contrôle de l'ancrage du stent 2a par rapport à la paroi intestinale 10.

Dans le mode de réalisation de la figure 3, le joint annulaire de colle élastomère 4a,4b à chaque extrémité longitudinale de la partie courante de l'élément d'ancrage 2, c'est-à-dire à chaque extrémité longitudinale 3a,3b de la gaine interne 3 et des première paroi 2a et deuxième paroi 2b, est constitué d'une première partie de joint annulaire 4-1a, 4-1b entre la gaine 3c et la deuxième paroi 2b et d'une deuxième partie de joint annulaire 4-2a, 4-2b entre les deux stents coaxiaux constituant la première paroi 2a et la deuxième paroi 2b.

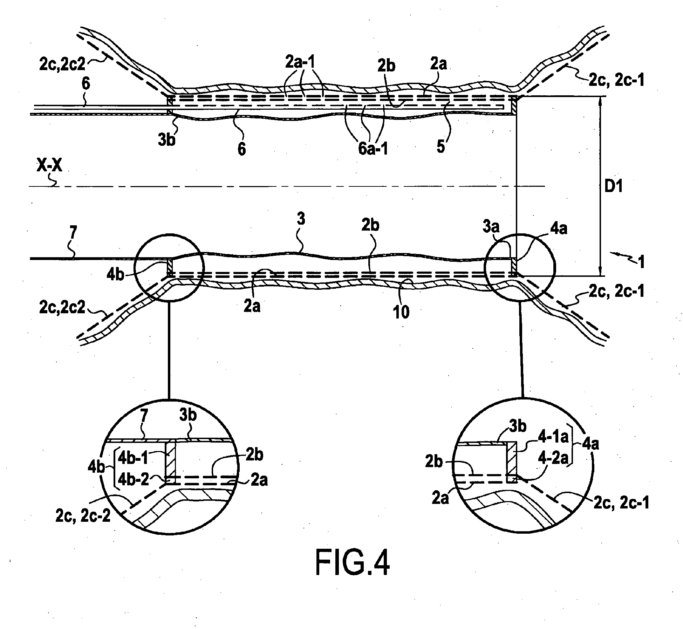

Sur la figure 4, on a représenté un mode de réalisation avec deux stents coaxiaux de diamètres nominaux de leurs parties courantes sensiblement identiques. Chaque stent comprend une collerette 2c, 2c-1, 2c-2 à une de ses extrémités longitudinales comme décrit ci-dessus et les deux stents 2a, 2b sont emboîtés coaxialement l'un dans l'autre tête bêche de sorte que l'élément d'ancrage 2 comprend une collerette à chaque extrémité longitudinale.

Du fait que les deux stents 2a, 2b présentent un diamètre nominal, c'est-à-dire un diamètre externe maximal D1, D2 sensiblement identique, le stent interne 2b a sa partie courante cylindrique constituant ladite deuxième paroi 2b ajustée en force contre la surface interne de la partie courante cylindrique du premier stent ou première paroi 2a. Et, les deux stents sont collés l'un à l'autre à leurs extrémités longitudinales de leurs dites parties courantes cylindriques ou première et deuxième parois 2a, 2b en vis-à-vis.

La gaine interne 3 est appliquée comme sur la figure 3 à l'intérieur du stent interne, collée à sa dite deuxième paroi 2b et la gaine interne 3 constitue ainsi la prolongation de la gaine externe 7.

Le tube d'injection-aspiration 6 est appliqué contre la surface interne de la deuxième paroi 2b comme sur la figure 3 entre la deuxième paroi 2b et la gaine interne 3.

Plus précisément, sur la figure 4, la deuxième paroi 2b ou stent interne présente à son extrémité amont une collerette 2c-1 et le stent externe ou première paroi 2a présente à son extrémité aval une collerette 2c-2, les deux première et deuxième parois 2a, 2b se chevauchant sur toute leur longueur.

Ainsi, du fait que la collerette avale 2c-2 est en prolongement du stent externe, il est possible de disposer le tube d'injection-aspiration 6 s'étendant en aval de l'élément d'ancrage 2 de telle sorte que son extrémité ouverte 6a débouche soit dans l'espace entre la gaine interne 3 et la deuxième paroi 2b du stent interne, comme représenté sur la figure 4, soit dans une variante non-représentée, avec l'extrémité ouverte du tube d'injection-aspiration 6 débouchant dans l'espace entre les deux stents. Plus précisément, la portion 6a du tube 6 peut être intercalée entre les deux première et deuxième parois 2a, 2b, c'est-à-dire collée sur la face externe de la deuxième paroi 2b.

Ce mode de réalisation de la figure 4 préserve un espace suffisant entre la gaine interne et la paroi intestinale 10, permettant ainsi d'éviter le colmatage des perforations 6a de l'extrémité du tube d'injection-aspiration 6. En outre, il confère une rigidité optimale à l'élément d'ancrage 2 et une stabilisation suffisamment longue dans la paroi intestinale avant sa migration après reprise du transit intestinal.

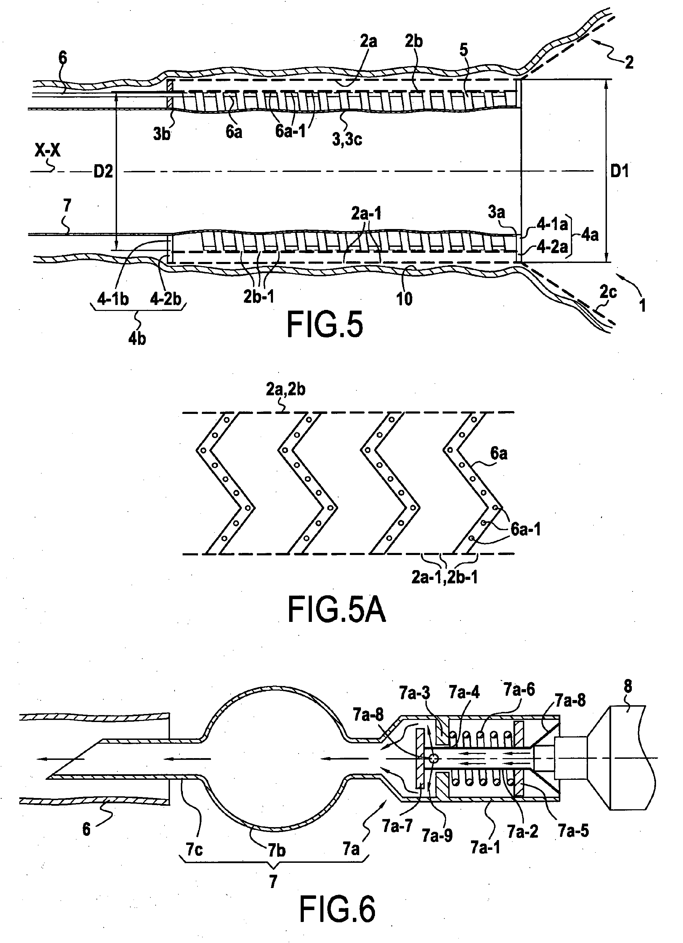

Sur la figure 5A, on a représenté schématiquement un mode de réalisation de l'extrémité du tube d'injection-aspiration 6 constitué par une portion de tube 6a multi-perforée 6a-1, s'étendant sensiblement hélicoïdalement contre la face interne de ladite deuxième paroi 2b comme représenté sur la figure 5, selon une ligne en zig-zag ou en Z en parcourant toute la longueur de la chambre de dépression 5. Ce type de tube en zig-zag est plaqué et collé contre la face interne de la deuxième paroi 2b.

La portion de tube multi-perforé 6a en zig-zag ou en Z au sein de la chambre 5 peut être également appliquée et collée sur la surface interne de la première paroi 2a ou la surface externe de la deuxième paroi 2b dans le mode de réalisation de la figure 2, en lui faisant parcourir hélicoïdalement la longueur dans la direction XX de la chambre annulaire 5.

Trois autres variantes du dispositif non représentées sont possibles :
1. stent 2a avec 2 collerettes et stent 2b sans collerette,
2. stent 2a sans collerette et stent 2b avec une seule collerette,
3. stent 2a sans collerette et stent 2b avec 2 collerettes.

Sur la figure 6, on a représenté l'extrémité longitudinale libre extérieure dudit tube d'injection-aspiration 6 connecté de façon réversible à un embout de raccordement 7 comportant un dispositif d'obturation consistant en une vanne anti-reflux 7a, comportant un dispositif témoin de la teneur du vide dans la chambre 5 consistant en un ballonnet7b.

Plus précisément, l'embout de raccordement 7 comporte une tige de raccordement rigide en 7c se prolongeant par ledit ballonnet témoin 7b lui-même coopérant avec et se prolongeant par le dispositif anti-reflux 7a.

L'embout de raccordement 7 peut être connecté à un appareil d'aspiration, par exemple une seringue 8 à l'extrémité libre du dispositif anti reflux 7a. Un embout de raccordement 7 de ce type est commercialisé par la société TELEFLEX MEDICAL COMPANY (USA) sous la référence RUSCHLIT PVC n° 103902035.

Le dispositif anti reflux 7a comporte un cylindre creux extérieur 7a-1 et un cylindre central creux interne 7a-2. Le cylindre central creux 7a-2 est guidé à l'intérieur du cylindre extérieur 7a-1 par une première butée 7a-3 solidaire de la paroi interne du cylindre extérieur 7a-1 et comportant un orifice cental 7a-4 à l'intérieur duquel passe ledit cylindre central creux 7a-2. Une deuxième butée 7a-5 est solidaire dudit cylindre central creux 7a-2 et un ressort 7a-6 est solidaire à chacune de ses extrémités d'un côté de ladite première butée 7a-3 et de l'autre côté à ladite deuxième butée 7a-5 de sorte que en position d'extension dudit ressort, une troisième butée 7a-7 solidaire de l'autre extrémité dudit cylindre central creux 7a-1 vient buter contre une face de la première butée 7a-3. Lorsque l'on adapte l'extrémité de la seringue 8 dans une extrémité évasée 7a-8 dudit cylindre central creux 7a-1, la force d'appui de la seringue permet de comprimer le ressort et de dégager lesdites première butée 7a-3 et troisième butée 7a-7 de sorte que l'air injecté par la seringue 8 peut s'écouler depuis un orifice 7a-8 à l'extrémité dudit cylindre central creux par le passage 7a-9 entre lesdites première butée 7a-3 et troisième butée 7a-7. Après le retrait de la seringue 8, le ressort 7a-6 reprend sa position d'extension et la troisième butée 7a-7 vient se plaquer contre la première butée 7a-3 et obturer tout passage d'air entre l'extérieur du dispositif anti reflux 7a et l'intérieur du ballonnet 7b.

En aspirant l'air dans le mini-tube perforé 6a avec la seringue 8 à travers l'embout de raccordement 7, on crée le vide dans la chambre de dépression 5. La muqueuse intestinale 10 est attirée à la surface externe du stent 2a sous l'effet de l'aspiration qui augmente l'ancrage du dispositif 1. Inversement, cet effet est annulé par injection d'air ou d'une solution liquide dans ladite chambre de dépression 5 au moyen du même mini-tube 6 depuis l'extrémité longitudinale libre extérieure du tube d'injection-aspiration 6.

Comme mentionné précédemment, lorsque l'on injecte une solution liquide dans la chambre de dépression 5, la température de la solution permet de modifier la température et donc la forme et la rigidité de la première paroi 2a réalisée en alliage de nitinol. Plus particulièrement aux températures situées en-dessous de 15°, le nitinol devient souple et malléable, de sorte que son réduction d'une solution liquide froide entre 0 et 15° dans la chambre 5 on peut rendre malléable le stent 2a et le cas échéant le stent 2b et faciliter leur migration lors du passage à travers l'anastomose ou d'une zone de rétrécissement par exemple.

Pour la réalisation des gaines souples 3 ou 7, on peut utiliser un mélange de différents types de silicone de type « LSR », « RTV » et « GEL », tels que décrits précédemment.

On a décrit dans WO 2010/09291 l'introduction du dispositif selon l'invention. Celui-ci est introduit sous forme rétractée par l'intermédiaire d'un introducteur 4 qui est constitué d'un tube en plastique semi rigide apte à être déformé de diamètre 3 à 20 mm, de préférence 10 à 15 mm, de longueur de 70 à 220 cm, au sein duquel l'élément d'ancrage est introduit sous forme rétractée, ladite gaine étant positionnée en aval de l'élément d'ancrage à l'intérieur du tube guide de l'introducteur. Une fois que le tube guide de l'introducteur est arrivé au site d'implantation, par exemple à environ 1 m en amont de l'anastomose, l'élément d'ancrage peut être sorti de l'extrémité de l'introducteur et il prend une position expansée. Il y a lieu de noter que le temps d'introduction et d'acheminement de l'introducteur de l'élément d'ancrage jusqu'au site d'implantation, est en pratique inférieur au temps à partir duquel ledit élément d'ancrage subit une expansion radiale de par l'augmentation de la température qu'il connaît à l'intérieur du corps.

Dans sa forme initiale, fermé et logé dans l'introducteur, le(s) stent(s) 2a,2b présente(nt) un diamètre très réduit notamment de 5 à 15 mm. On traverse l'anastomose, puis on pénètre l'intestin d'amont. Le chirurgien apprécie la progression de l'introducteur et son bon emplacement par la palpation de l'introducteur à travers les parois de l'intestin et en visualisant le stent pendant son expansion. Une fois libérée dans la lumière de l'intestin, le stent retrouve progressivement son diamètre définitif. Il peut être temporairement maintenu en place par le chirurgien, qui pince le stent à travers les parois de l'intestin. L'introducteur est ensuite retiré. La gaine 7 est dépliée spontanément et progressivement au retrait de l'introducteur. Ce dernier retraverse l'anastomose puis l'orifice anal dans le sens antégrade, libérant totalement la gaine 7. Après un délai moyen de 4 à 6 jours, et sous l'effet des contractions intestinales, l'unité stent-gaine externe- tube d'injection-aspiration migre progressivement vers l'orifice anal depuis le site d'ancrage en amont, lequel est suffisamment en amont de préférence à au moins 20 cm de longueur d'intestin de l'anastomose pour que le stent atteigne l'orifice anal 5 ou 6 jours plus tard seulement après reprise du transit intestinal, puis, le dispositif est éliminé avec la matière fécale.

Le dispositif selon l'invention peut être combiné à d'autres moyens en vue d'un autre usage que la protection d'une anastomose.

En particulier, il peut être utilisé pour tous les usages requérant un ancrage dans l'intestin, notamment en vue de mieux contrôler cet ancrage.

On cite notamment les dispositifs visant à l'augmentation de la sensation de satiété tel que décrit dans US 2008/0208357.

## Revendications

1. Dispositif chirurgical (1) d'ancrage, apte à s'ancrer sur la muqueuse de la paroi interne de l'intestin (10), comprenant un élément d'ancrage temporaire (2) dont l'ancrage peut être modifié de façon contrôlée, comprenant au moins un premier élément longitudinal creux semi-rigide, définissant une paroi de révolution autour d'un axe longitudinal (XX) comprenant une partie courante multi-perforée sensiblement cylindrique à section sensiblement circulaire dite première paroi (2a), ledit premier élément longitudinal creux étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre :
- un premier diamètre externe minimal (D1') en dite position radiale rétractée de ladite première paroi, d'au plus 20 mm, de préférence d'au plus 10mm, et
- un premier diamètre externe maximal (D1) en dite position d'expansion radiale maximale de ladite première paroi, de préférence de 18 à 45 mm,
**caractérisé en ce qu'**au moins une partie, de préférence toute la longueur, de la surface interne cylindrique de ladite première paroi (2a) est doublée d'une couche étanche indépendante formant une gaine interne (3), seules les extrémités longitudinales (3a,3b) de ladite gaine interne (3) étant fixées de manière étanche (4a,4b) audit élément d'ancrage (2) à l'aide des premiers moyens de fixation étanche (4-1a,4-1b), de préférence un joint annulaire de colle élastomère, à chaque dite extrémité longitudinale de ladite gaine interne, de façon à délimiter une chambre dénommée chambre de dépression (5) entre ladite gaine interne et ladite première paroi, ledit élément d'ancrage temporaire étant couplé à un tube souple ou semi rigide dénommé tube d'injection-aspiration (6), s'étendant à l'extérieur dudit élément d'ancrage (2), une extrémité ouverte (6a) dudit tube d'injection-aspiration débouchant dans ladite chambre de dépression (5).

2. Dispositif selon la revendication 1, **caractérisé en ce que** ladite gaine interne (3) est constituée d'un film étanche (3c) formant une paroi tubulaire souple.

3. Dispositif selon la revendication 1 ou 2, **caractérisé en ce que** l'extrémité (6a) dudit tube d'injection-aspiration débouchant dans ladite chambre est constituée d'une portion de tube multi-perforé s'étendant dans ladite chambre (5) de préférence sensiblement sur toute sa longueur de ladite chambre, de préférence encore sous forme d'une ligne en Z en parcourant sensiblement hélicoïdalement ladite chambre de dépression collée contre la surface interne dudit élément d'ancrage.

4. Dispositif selon l'une des revendications 1 à 3, **caractérisé en ce que** l'extrémité dudit tube d'injection-aspiration (6) débouchant dans ladite chambre de dépression (5) traverse de façon étanche un desdits premiers moyens de fixation, de préférence un dit joint annulaire de colle (4-1b), à une extrémité longitudinale de ladite gaine interne.

5. Dispositif selon l'une des revendications 1 à 4, **caractérisé en ce qu'**il comprend en outre une gaine externe (7) à paroi tubulaire constituée d'un film souple fixée audit élément d'ancrage (2) à une extrémité longitudinale dudit élément d'ancrage.

6. Dispositif selon la revendication 5, **caractérisé en ce que** ladite gaine externe (7) constitue un prolongement de ladite gaine interne (3) s'étendant à l'extérieur dudit élément d'ancrage.

7. Dispositif selon la revendication 5 ou 6, **caractérisé en ce que** ledit tube d'injection-aspiration (6) et ladite gaine externe (7) s'étendent à l'extérieur dudit élément d'ancrage depuis la même extrémité dudit élément d'ancrage et de préférence ledit tube d'injection-aspiration (6) et ladite gaine externe (7) sont solidarisés entre eux, ledit tube d'injection-aspiration (6) étant situé à l'extérieur ladite gaine externe (7).

8. Dispositif selon l'une des revendications 1 à 7, **caractérisé en ce que** ledit élément d'ancrage comporte un deuxième élément longitudinal creux semi-rigide, définissant une paroi de révolution autour dudit axe longitudinal (XX) comprenant une partie courante multi-perforée sensiblement cylindrique à section sensiblement circulaire dite deuxième paroi (2b), ladite deuxième paroi étant disposée co axialement à l'intérieur de ladite première paroi (2a), les extrémités longitudinales de ladite deuxième paroi (2b) étant fixées de manière étanche à celles de ladite première paroi (2a) à l'aide de premiers (4-1a,4-1b) ou deuxièmes moyens de fixation étanches (4-2a,4-2b), de préférence un joint annulaire de colle élastomère (4a,4b) à chaque dite extrémité longitudinale de ladite deuxième paroi, ledit deuxième élément longitudinal creux étant réalisé en un matériau lui conférant des propriétés d'élasticité radiale de sorte qu'il peut être comprimé radialement en dite position rétractée et adopter une dite position d'expansion radiale maximale après relâchement de la compression radiale de telle sorte que ledit deuxième diamètre externe de ladite deuxième paroi (2b) puisse varier de façon contrôlée entre :
- un deuxième diamètre externe minimal (D2') en position radiale rétractée de ladite deuxième paroi, inférieur audit premier diamètre externe minimal, et
- un deuxième diamètre externe maximal (D2) en position d'expansion radiale maximale de ladite deuxième paroi, inférieur audit premier diamètre externe maximal.

9. Dispositif selon la revendication 8, **caractérisé en ce que** ladite deuxième paroi multi-perforée (2b) est intercalée entre ladite gaine interne (3) et ladite première paroi (2a), l'extrémité dudit tube d'injection-aspiration (6a) débouchant de préférence dans ladite chambre de dépression entre ladite gaine interne(3) et ladite deuxième paroi (2b).

10. Dispositif selon l'une des revendications 1 à 9, **caractérisé en ce que** ladite première paroi (2a) présente :
a) une longueur (L1) d'au moins 30 mm, de préférence de 40 à 150 mm, et un diamètre externe que l'on peut faire varier de façon contrôlée entre un diamètre externe minimal (D1') en position radiale rétractée de ladite première paroi d'au plus 10mm, et un diamètre externe maximal (D1) en position d'expansion radiale maximale de ladite première paroi, de 18 à 45 mm, de préférence de 20 à 35 mm, et
b) de préférence, au moins une de ses extrémités longitudinales, de préférence encore son extrémité amont, avec une prolongation évasée (2c) dénommée collerette, définissant une paroi de révolution autour dudit axe longitudinal (XX) à section circulaire de diamètre croissant, de préférence de 5 à 30mm de longueur (L1'), de préférence encore de 15 à 20 mm de longueur, et de diamètre maximal (D0) égal à au moins 105% du dit premier diamètre maximal de préférence de 105 à 150%.

11. Dispositif selon l'une des revendications 8 à 10, **caractérisé en ce que** ledit deuxième élément longitudinal creux comporte une deuxième paroi (2b) ajustée contre la surface interne de ladite première paroi (2a), lesdites première et deuxième parois étant de préférence de même longueur.

12. Dispositif selon l'une des revendications 9 à 11, **caractérisé en ce que** chaque dit premier et second élément longitudinal creux comporte à une des extrémités longitudinales de chaque dite première et respectivement deuxième paroi (2a, 2b) une prolongation évasée (2c, 2c-1, 2c-2) dénommée collerette, définissant une surface de révolution autour dudit axe longitudinal (XX) à section circulaire de diamètre croissant depuis l'extrémité de ladite première ou respectivement deuxième paroi vers l'extrémité dudit premier ou respectivement deuxième élément longitudinal creux, et de diamètre maximal (D0) égal à au moins 105% dudit premier diamètre maximal (D1) de préférence de 105 à 150%, les deux dits élément longitudinaux creux étant emboités tête-bêche de sorte que ledit élément d'ancrage comporte une dite collerette à chaque extrémité longitudinale.

13. Dispositif selon l'une des revendications 1 à 12, **caractérisé en ce que** ledit élément d'ancrage (2,2a,2b) comporte une collerette (2c,2c-1,2c-2), de préférence une collerette à chaque extrémité, et un fil dénommé lasso est accroché en bordure de ladite collerette , de préférence l'un au moins des deux fils étant d'une longueur au moins égale à celle du dit tube d'injection-aspiration et le cas échéant de ladite gaine externe.

14. Dispositif selon l'une des revendications 1 à 13, **caractérisé en ce que** lesdites gaine interne (3) et gaine externe (7) sont en matériau synthétique biocompatible, d'épaisseur de paroi de 0,01 à 1 mm, de préférence en matériaux élastomère du type silicone ou polyuréthane de 0,05 à 1 mm d'épaisseur, et de préférence encore présentant des propriétés d'élasticité radiale et longitudinale, et ladite gaine externe au moins présentant des propriétés de mémoire de forme et des propriétés de non collabilité, ladite gaine externe (7) présentant au repos une longueur (L2) en aval dudit élément d'ancrage (2) d'au moins 50 cm, de préférence au moins 1 m, et un diamètre externe de 18 à 45 mm, de préférence de 20 à 35 mm.

15. Dispositif selon l'une des revendications 1 à 14, **caractérisé en ce que** ledit tube d'injection-aspiration (6) est connecté à son extrémité longitudinale libre à un embout de raccordement (7), lui-même connecté de façon réversible ou apte à être connecté de façon réversible avec un dispositif d'injection ou aspiration d'air ou liquide tel qu'une seringue (8), ledit embout de raccordement comprenant un dispositif d'obturation, de préférence une vanne anti-reflux (7a) et un dispositif de témoin de vide apte à indiquer la teneur du vide dans ladite chambre de dépression (5) à l'autre extrémité (6a) dudit tube d'injection-aspiration, ledit dispositif témoin étant de préférence un ballonnet (7b).

16. Dispositif selon l'une des revendications 1 à 15, **caractérisé en ce que** ledit élément d'ancrage (2) temporaire est constitué de prothèse(s) entérale(s) de type stent formé par un maillage de fils spiralés métalliques ou en élastomère, de préférence en nitinol.

## Patentansprüche

1. Chirurgische Verankerungsvorrichtung (1), die geeignet ist, sich in der Schleimhaut der Innenwand des Darms (10) zu verankern, umfassend ein temporäres Verankerungselement (2), dessen Verankerung auf kontrollierte Weise verändert werden kann, umfassend mindestens ein erstes halbsteifes hohles Längselement, das eine Umdrehungswand um eine Längsachse (XX) definiert, umfassend einen mehrfach perforierten im Wesentlichen zylindrischen Laufteil mit im Wesentlichen kreisförmigem Querschnitt, erste Wand (2a) genannt, wobei das erste hohle Längselement aus einem Material hergestellt ist, das ihm radiale Elastizitätseigenschaften verleiht, so dass es radial in eine zusammengezogene Position komprimiert werden kann, und nach dem Lösen der radialen Kompression eine sogenannte Position maximaler radialer Expansion einnehmen kann, so dass die erste mehrfach perforierte Wand einen ersten Außendurchmesser aufweist, der auf kontrollierte Weise variiert werden kann zwischen:
- einem ersten minimalen Außendurchmesser (D1') in der zusammengezogenen Position der ersten Wand von höchstens 20 mm, vorzugsweise höchstens 10 mm, und
- einem ersten maximalen Außendurchmesser (D1) in der Position maximaler radialer Expansion der ersten Wand von vorzugsweise 18 bis 45 mm,
**dadurch gekennzeichnet, dass** mindestens ein Teil, vorzugsweise die gesamte Länge der zylindrischen Innenfläche der ersten Wand (2a) mit einer unabhängigen dichten Schicht, die eine innere Hülle (3) bildet, verdoppelt ist, wobei nur die Längsenden (3a, 3b) der inneren Hülle (3) dicht (4a, 4b) am Verankerungselement (2) mit Hilfe der ersten Mittel zur dichten Befestigung (4-1 a, 4-1 b), vorzugsweise einer Ringdichtung aus Elastomerkleber, an jedem Längsende der inneren Hülle befestigt sind, um eine so genannte Unterdruckkammer (5) zwischen der inneren Hülle und der ersten Wand zu begrenzen, wobei das Element zur temporären Verankerung an ein biegsames oder halbsteifes Rohr, Einspritz-Ansaugrohr (6) genannt, gekoppelt ist, das sich außerhalb des Verankerungselements (2) erstreckt, wobei ein offenes Ende (6a) des Einspritz-Ansaugrohrs in die Unterdruckkammer (5) mündet.

2. Vorrichtung nach Anspruch 1, **dadurch gekennzeichnet, dass** die innere Hülle (3) von einer dichten Folie (3c) gebildet ist, die eine biegsame röhrenförmige Wand bildet.

3. Vorrichtung nach Anspruch 1 oder 2, **dadurch gekennzeichnet, dass** das Ende (6a) des Einspritz-Ansaugrohrs, das in die Kammer mündet, von einem mehrfach perforierten Rohrabschnitt gebildet ist, der sich in der Kammer (5) vorzugsweise im Wesentlichen über die gesamte Länge der Kammer erstreckt, ferner vorzugsweise in Form einer Z-förmigen Linie, die im Wesentlichen spiralförmig die Kammer durchläuft und an die Innenfläche des Verankerungselements geklebt ist.

4. Vorrichtung nach einem der Ansprüche 1 bis 3, **dadurch gekennzeichnet, dass** das Ende des Einspritz-Ansaugrohrs (6), das in die Unterdruckkammer (5) mündet, dicht durch eines der ersten Befestigungsmittel, vorzugsweise eine Ringdichtung aus Kleber (4-1 b), an einem Längsende der inneren Hülle hindurchgeht.

5. Vorrichtung nach einem der Ansprüche 1 bis 4, **dadurch gekennzeichnet, dass** sie ferner eine äußere Hülle (7) mit einer röhrenförmigen Wand umfasst, die von einer flexiblen Folie, die am Verankerungselement (2) an einem Längsende des Verankerungselements befestigt ist, gebildet ist.

6. Vorrichtung nach Anspruch 5, **dadurch gekennzeichnet, dass** die äußere Hülle (7) eine Verlängerung der inneren Hülle (3) darstellt, die sich außerhalb des Verankerungselements erstreckt.

7. Vorrichtung nach Anspruch 5 oder 6, **dadurch gekennzeichnet, dass** sich das Einspritz-Ansaugrohr (6) und die äußere Hülle (7) außerhalb des Verankerungselements von demselben Ende des Verankerungselements aus erstrecken, und dass vorzugsweise das Einspritz-Ansaugrohr (6) und die äußere Hülle (7) miteinander verbunden sind, wobei sich das Einspritz-Ansaugrohr (6) außerhalb der äußeren Hülle (7) befindet.

8. Vorrichtung nach einem der Ansprüche 1 bis 7, **dadurch gekennzeichnet, dass** das Verankerungselement ein erstes halbsteifes hohles Längselement umfasst, das eine Umdrehungswand um eine Längsachse (XX) definiert, umfassend einen mehrfach perforierten im Wesentlichen zylindrischen Laufteil mit im Wesentlichen kreisförmigem Querschnitt, zweite Wand (2b) genannt, wobei die zweite Wand koaxial innerhalb der ersten Wand (2a) angeordnet ist, wobei die Längsenden der zweiten Wand (2b) dicht en jenen der ersten Wand (2a) mit Hilfe von ersten (4-1 a, 4-1 b) oder zweiten Mitteln zur dichten Befestigung (4-2a, 4-2b), vorzugsweise einer Ringdichtung aus Elastomerkleber (4a, 4b) an jedem Längsende der zweiten Wand befestigt sind, wobei das zweite hohle Längselement aus einem Material hergestellt ist, das ihm radiale Elastizitätseigenschaften verleiht, so dass es radial in die zusammengezogene Position komprimiert werden kann, und nach dem Lösen der radialen Kompression eine sogenannte Position maximaler radialer Expansion einnehmen kann, so dass der zweite Außendurchmesser der zweiten Wand (2b) auf kontrollierte Weise variieren kann zwischen:
- einem zweiten minimalen Außendurchmesser (D2') in der zusammengezogenen Position der zweiten Wand, der kleiner als der erste minimale Außendurchmesser ist, und
- einem zweiten maximalen Außendurchmesser (D2) in der Position maximaler radialer Expansion der zweiten Wand, der kleiner als der erste maximale Außendurchmesser ist.

9. Vorrichtung nach Anspruch 8, **dadurch gekennzeichnet, dass** die zweite mehrfach perforierte Wand (2b) zwischen der inneren Hülle (3) und der ersten Wand (2a) angeordnet ist, wobei das Ende des Einspritz-Ansaugrohrs (6a) vorzugsweise in die Unterdruckkammer zwischen der inneren Hülle (3) und der zweiten Wand (2b) mündet.

10. Vorrichtung nach einem der Ansprüche 1 bis 9, **dadurch gekennzeichnet, dass** die erste Wand (2a) folgendes aufweist:
a) eine Länge (L1) von mindestens 30 mm, vorzugsweise von 40 bis 150 mm, und einen Außendurchmesser, der auf kontrollierte Weise zwischen einem minimalen Außendurchmesser (D1') in der zusammengezogenen radialen Position der ersten Wand von höchstens 10 mm und einem maximalen Außendurchmesser (D1) in der Position maximaler radialer Expansion der ersten Wand von 18 bis 45 mm, vorzugsweise von 20 bis 35 mm, variiert werden kann, und
b) vorzugsweise mindestens eines ihrer Längsenden, ferner vorzugsweise ihr stromaufwärtiges Ende, mit einer erweiterten Verlängerung (2c), Kragen genannt, die eine Umdrehungswand um die Längsachse (XX) mit kreisförmigem Querschnitt mit steigendem Durchmesser, vorzugsweise von 5 bis 30 mm Länge (all'), ferner vorzugsweise von 15 bis 20 mm Länge, und mit einem maximalen Durchmesser (D0) gleich mindestens 105 % des ersten maximalen Durchmessers von 105 bis 150 % definiert.

11. Vorrichtung nach einem der Ansprüche 8 bis 10, **dadurch gekennzeichnet, dass** das zweite hohle Längselement eine zweite Wand (2b) umfasst, die gegen die Innenfläche der ersten Wand (2a) angepasst ist, wobei die erste und die zweite Wand vorzugsweise dieselbe Länge haben.

12. Vorrichtung nach einem der Ansprüche 9 bis 11, **dadurch gekennzeichnet, dass** jedes erste und zweite hohle Längselement an einem der Längsenden jeder ersten bzw. zweiten Wand (2a, 2b) eine erweiterte Verlängerung (2c, 2c-1, 2c-2), Kragen genannt, aufweist, die eine Umdrehungsfläche um die Längsachse (XX) mit kreisförmigem Querschnitt mit einem steigenden Durchmesser vom Ende der ersten bzw. zweiten Wand bis zum Ende des ersten bzw. zweiten hohlen Längselements und mit einem maximalen Durchmesser (D0) gleich mindestens 105 % des ersten maximalen Durchmessers (D1) vorzugsweise von 105 bis 150 %, definiert, wobei die beiden hohlen Längselemente gegensinnig ineinandergesteckt sind, so dass das Verankerungselement einen Kragen an jedem Längsende umfasst.

13. Vorrichtung nach einem der Ansprüche 1 bis 12, **dadurch gekennzeichnet, dass** das Verankerungselement (2, 2a, 2b) einen Kragen (2c, 2c-1, 2c-2), vorzugsweise einen Kragen an jedem Ende, umfasst, und ein so genannter Lasso-Faden am Rand des Kragens befestigt ist, wobei vorzugsweise mindestens einer der Fäden eine Länge mindestens gleich jener des Einspritz-Ansaugrohrs und gegebenenfalls der äußeren Hülle hat.

14. Vorrichtung nach einem der Ansprüche 1 bis 13, **dadurch gekennzeichnet dass** die innere Hülle (3) und die äußere Hülle (7) aus einem biologisch abbaubaren synthetischen Material mit einer Wandstärke von 0,01 bis 1 mm sind, vorzugsweise aus Elastomermaterialien des Typs Silikon oder Polyurethan mit einer Dicke von 0,05 bis 1 mm, die ferner vorzugsweise in Radial- und Längsrichtung elastische Eigenschaften aufweisen, wobei mindestens die äußere Hülle Formgedächtniseigenschaften und nicht klebende Eigenschaften aufweist, wobei die äußere Hülle (7) in Ruheposition eine Länge (L2) stromabwärts zum Verankerungselement (2) von mindestens 50 cm, vorzugsweise mindestens 1 m, und einen Außendurchmesser von 18 bis 45 mm, vorzugsweise 20 bis 35 mm, aufweist.

15. Vorrichtung nach einem der Ansprüche 1 bis 14, **dadurch gekennzeichnet, dass** das Einspritz-Ansaugrohr (6) an seinem freien Längsende an einen Anschlussstutzen (7) angeschlossen ist, welcher selbst an eine Vorrichtung zum Einspritzen oder Ansaugen von Luft oder Flüssigkeit, wie eine Spritze (8), angeschlossen oder geeignet ist, angeschlossen zu werden, wobei der Anschlussstutzen eine Verschlussvorrichtung, vorzugsweise ein Rückschlagventil (7a), und eine Vorrichtung zur Vakuumanzeige umfasst, die geeignet ist, den Gehalt des Vakuums in der Unterdruckkammer (5) des anderen Endes (6a) des Einspritz-Ansaugrohrs anzuzeigen, wobei die Anzeigevorrichtung vorzugsweise ein Ballon (7b) ist.

16. Vorrichtung nach einem der Ansprüche 1 bis 15, **dadurch gekennzeichnet, dass** das temporäre Verankerungselement (2) von Enteralprothesen vom Typ Stent gebildet ist, welche von einem Netzwerk von Spiralfäden aus Metall oder Elastomer, vorzugsweise aus Nitinol, gebildet ist.

## Claims

1. A surgical anchor device (1) suitable for being anchored in the mucous membrane of the inner wall of the intestine (10), the device comprising a temporary anchor element (2) of anchoring that can be modified in controlled manner, comprising at least a first semi-rigid hollow longitudinal element defining a wall in the form of a surface of revolution about a longitudinal axis (XX) having a substantially cylindrical multiply-perforated main portion of substantially circular section referred to as a "first" wall (2a), said first hollow longitudinal element being made of a material that gives it properties of radial elasticity such that it can be compressed radially into a retracted position and can adopt a said position referred to as a maximally radially expanded position after the radial compression has been released, whereby said first multiply-perforated wall presents a first outer diameter that can be varied in controlled manner between:
• a minimum first outer diameter (D1') in said radially retracted position of said first wall of at most 20 mm, preferably of at most 10 mm; and
• a maximum first outer diameter (D1) in said maximally radially expanded position of said first wall, preferably lying in the range 18 mm to 45 mm;
the device being **characterized in that** at least a portion and preferably all of the length of the cylindrical inner surface of said first wall (2a) is lined with an independent leakproof layer forming an inner sheath (3), only the longitudinal ends (3a, 3b) of said inner sheath (3) being fastened in leaktight manner (4a, 4b) to said anchor element (2) with the help of first leaktight fastener means (4-1a, 4-1b), preferably respective annular elastomer adhesive gaskets, at each of said longitudinal ends of said inner sheath, so as to define a chamber referred to as a suction chamber (5) between said inner sheath and said first wall, said temporary anchor element being coupled to a flexible or semi-rigid tube referred to as an injection-suction tube (6) extending outside said anchor element (2), an open end (6a) of said injection-suction tube opening out into said suction chamber (5).

2. A device according to claim 1, **characterized in that** said inner sheath (3) is constituted by a leakproof film (3c) forming a flexible tubular wall.

3. A device according to claim 1 or claim 2, **characterized in that** the end (6a) of said injection-suction tube opening out into said chamber is constituted by a multiply-perforated tube portion extending inside said chamber (5), preferably substantially over the entire length of said chamber, also preferably in the form of a Z-shaped line running substantially helically along said suction chamber and stuck to the inner surface of said anchor element.

4. A device according to any one of claims 1 to 3, **characterized in that** the end of said injection-suction tube (6) opening out into said suction chamber (5) passes in leaktight manner through one of said first fastener means, preferably a said annular adhesive gasket (4-1b), at one of the longitudinal ends of said inner sheath.

5. A device according to any one of claims 1 to 4, **characterized in that** it further includes an outer sheath (7) having a tubular wall constituted by a flexible film fastened to said anchor element (2) at one of the longitudinal ends of said anchor element.

6. A device according to claim 5, **characterized in that** said outer sheath (7) constitutes an extension of said inner sheath (3) extending outside said anchor element.

7. A device according to claim 5 or claim 6, **characterized in that** said injection-suction tube (6) and said outer sheath (7) extend outside said anchor element from the same end of said anchor element, and preferably said injection-suction tube (6) and said outer sheath (7) are secured to each other, said injection-suction tube (6) being situated outside said outer sheath (7).

8. A device according to any one of claims 1 to 7, **characterized in that** said anchor element includes a second semi-rigid hollow longitudinal element defining a wall in the form of a surface of revolution around said longitudinal axis (XX) having a substantially cylindrical multiply-perforated main portion of substantially circular section referred to as a "second" wall (2b), said second wall being arranged coaxially inside said first wall (2a), the longitudinal ends of said second wall (2b) being fastened in leaktight manner to the ends of said first wall (2a) with the help of first or second leaktight fastener means (4-1a, 4-1b; 4-2a, 4-2b), preferably respective annular elastomer adhesive gaskets (4a, 4b) at each of said longitudinal ends of said second wall, said second hollow longitudinal element being made of a material giving it properties of radial elasticity such that it can be compressed radially into a said retracted position and adopt a said maximally radial expanded position such that said second outer diameter of said second wall (2b) can vary in controlled manner between:
• a minimum second outer diameter (D2') in the radially retracted position of said second wall that is less than said minimum first outer diameter; and
• a maximum second outer diameter (D2) in the maximally radially expanded position of said second wall that is less than said maximum first outer diameter.

9. A device according to claim 8, **characterized in that** said second multiply-perforated wall (2b) is interposed between said inner sheath (3) and said first wall (2a), the end of said injection-suction tube (6a) preferably opening out into said suction chamber between said inner sheath (3) and said second wall (2b).

10. A device according to any one of claims 1 to 9, **characterized in that** said first wall (2a) presents:
a) a length (L1) of at least 30 mm, preferably lying in the range 40 mm to 150 mm, and an outer diameter that may be varied in controlled manner between a minimum outer diameter (D1') in the radially retracted position of said first wall of no more than 10 mm, and a maximum outer diameter (D1) in the maximally radially expanded position of said first wall lying in the range 18 mm to 45 mm, preferably in the range 20 mm to 35 mm; and
b) preferably, at least one of its longitudinal ends, more preferably its upstream end, having a flared extension (2c) referred to as a "collar", defining a wall in the form of a surface of revolution about said longitudinal axis (XX) with a circular section of increasing diameter, having a length (L1') preferably lying in the range 5 mm to 30 mm, more preferably in the range 15 mm to 20 mm, and a maximum diameter (D0) equal to at least 105% of said maximum first diameter and preferably lying in the range 105% to 150%.

11. A device according to any one of claims 8 to 10, **characterized in that** said second hollow longitudinal element includes a second wall (2b) fitted against the inner surface of said first wall (2a), said first and second walls preferably being of the same length.

12. A device according to any one of claims 9 to 11, **characterized in that** each said first and second hollow longitudinal element includes at one of the longitudinal ends of each said first and second walls (2a, 2b) respectively a flared extension (2c, 2c-1, 2c-2) referred to as a "collar", defining a surface of revolution about said longitudinal axis (XX) with a circular section of diameter that increases from the end of said first or second wall towards the end of the corresponding said first or second hollow longitudinal element, and of maximum diameter (D0) equal to at least 105% of said maximum first diameter (D1) and preferably lying in the range 105% to 150%, said two hollow longitudinal elements being engaged one inside the other and head to tail so that said anchor element has a said collar at each of its longitudinal ends.

13. A device according to any one of claims 1 to 12, **characterized in that** said anchor element (2, 2a, 2b) includes a collar (2c, 2c-1, 2c-2), preferably a collar at each end, and a yarn referred to as a "lasso" is attached to the margin of said collar, with preferably at least one of the two yarns being of a length not less than the length of said injection-suction tube, and where appropriate of said outer sheath.

14. A device according to any one of claims 1 to 13, **characterized in that** said inner and outer sheaths (3, 7) are made of biocompatible synthetic material, having a wall thickness of 0.01 mm to 1 mm, preferably being made of silicone or polyurethane type elastomer materials having thickness in the range 0.05 mm to 1 mm, and more preferably presenting properties of radial and longitudinal elasticity, and at least said outer sheath presenting shape memory properties and non-stick properties, said outer sheath (7) at rest presenting a length (L2) downstream from said anchor element (2) of at least 50 cm, preferably of at least 1 m, and an outer diameter lying in the range 18 mm to 45 mm, preferably lying in the range 20 mm to 35 mm.

15. A device according to any one of claims 1 to 14, **characterized in that** said injection-suction tube (6) is connected at its free longitudinal end to a connection endpiece (7), itself connected reversibly or suitable for being connected reversibly with a device such as a syringe (8) for injecting or sucking air or liquid, said connection endpiece including a shutter device, preferably an anti-reflux valve (7a), and a vacuum indicator device suitable for indicating the degree of vacuum in said suction chamber (5) at the other end (6a) of said injection-suction tube, said indicator device preferably being a balloon (7b).

16. A device according to any one of claims 1 to 15, **characterized in that** said temporary anchor element (2) is constituted by one or more enteral prostheses of the stent type formed by a mesh of spiral-wound metal or elastomer wires or yarns, preferably of nitinol wires.
